# EUROPEAN PATENT SPECIFICATION

(11) **EP 0 967 261 B1**
(45) Date of publication and mention of the grant of the patent: **02.04.2003**
(21) Application number: 98116112.8
(22) Date of filing: 26.08.1998
(51) Int. Cl.: C09K 19/34, C07D 309/04, C09K 19/04

(54) **Liquid crystal compound having negative dielectric anisotropy, liquid crystal composition containing said liquid crystal compound and liquid crystal display device using said composition**
Flüssigkristallverbindung mit negativer dielektrischer Anisotropie, Flüssigkristallzusammensetzung enthaltend diese Verbindung und Flüssigkristallanzeigevorrichtung verwendend diese Zusammensetzung
Composé liquide cristallin à anisotropie diélectrique négative, composition liquide cristalline contenant ce composé liquide cristallin et dispositif d'affichage à cristaux liquides utilisant cette composition

(30) Priority: 25.06.1998 JP 19501898
(43) Date of publication of application: 29.12.1999
(73) Proprietor: Chisso Corporation, Osaka-shi Osaka 530-0055 (JP)
(72) Inventor: Yamada, Keizo, Chiba-shi, Chiba-ken (JP); Yano, Hitoshi, Ichihara-shi, Chiba-ken (JP); Kondo, Tomoyuki, Ichihara-shi, Chiba-ken (JP)
(74) Representative: Müller-Boré & Partner Patentanwälte

(56) References cited:
- EP-A- 0 117 476
- DONG C C ET AL: "THE SYNTHESIS AND TRANSITION TEMPERATURES OF SOME FLUORINATED TERPHENYLS WITH ALKENYL TERMINAL CHAINS" FERROELECTRICS, vol. 180, 23 July 1995 (1995-07-23), pages 245-257, XP002050858
- CHEMICAL ABSTRACTS, vol. 123, no. 19, 6 November 1995 (1995-11-06) Columbus, Ohio, US; abstract no. 256748, AZUMAI, TAKAYUKI ET AL: "Preparation of heterocycle-containing acetylene alcohol derivatives as intermediates for pharmaceuticals, agrochemicals, and liquid crystals" XP002114699 & JP 07 041442 A (SUMITOMO CHEMICAL CO, JAPAN) 1993
- CHEMICAL ABSTRACTS, vol. 123, no. 21, 20 November 1995 (1995-11-20) Columbus, Ohio, US; abstract no. 286064, AZUMAI, TAKAYUKI ET AL: "Preparation of pyrimidine derivatives and analogs as intermediates for liquid crystals, pharmaceuticals, and agrochemicals" XP002114700 & JP 07 061942 A (SUMITOMO CHEMICAL CO, JAPAN) 1993
- CHEMICAL ABSTRACTS, vol. 123, no. 23, 4 December 1995 (1995-12-04) Columbus, Ohio, US; abstract no. 314002, FUJIMOTO, YUKARI ET AL: "Method for producing phenol, heterocyclylphenol, and heterocycle-condense phenol derivatives, and related compounds" XP002114701 & JP 07 118241 A (SUMITOMO CHEMICAL CO, JAPAN) 1994
- PATENT ABSTRACTS OF JAPAN vol. 098, no. 014, 31 December 1998 (1998-12-31) & JP 10 237448 A (CHISSO CORP), 8 September 1998 (1998-09-08)
- DATABASE WPI Section Ch, Week 9835 Derwent Publications Ltd., London, GB; Class E13, AN 98-408672 XP002114702 & JP 10 168076 A (CHISSO CORP), 23 June 1998 (1998-06-23)

## Description

The present invention mainly relates to a novel liquid crystal compound having characteristic properties favorable for a liquid crystal composition used for liquid crystal display devices of various display modes such as vertically aligned mode, IPS (in-plane switching) mode, TFT (thin film transistor) mode, TN (twisted nematic) mode or STN (super twisted nematic) mode, in particular, vertically aligned mode and IPS display mode; a liquid crystal composition having excellent physical properties; and a liquid crystal display device produced using the liquid crystal composition. In the present invention, the term "liquid crystal compound" is a general term for compounds capable of exhibiting liquid crystal phases and compounds which do not exhibit any liquid crystal phase, but are useful as ingredients for liquid crystal compositions.

Up to this time, there have been proposed various liquid crystal display modes. As an example thereof, there can be listed the following display mode (see "Up-To-Date Liquid Crystal Technique", edited by Kogyochosa Kai, 1983). Examples of modes which make use of liquid crystal compositions having positive dielectric anisotropy are TN modes, STN modes, or TN based AM (active matrix) mode (such as TFT or MIM (metal-insulating film-metal) modes). Moreover, as modes which make use of liquid crystal compositions having negative dielectric anisotropy, there may be listed, for instance, ECB (Electric field-control birefringent effect) modes (such as HAN (hybrid molecular arrangement) or DAP (vertical molecular arrangement) mode), DS (dynamic scattering) modes, GH (guest-host) modes or PC (phase transition) modes.

Among these modes, the mainstreams thereof which have presently been used are those which make use of liquid crystal compositions having positive dielectric anisotropy. Contrary to this, there has been a delay in the practical application of the modes utilizing liquid crystal compositions having negative dielectric anisotropy. In connection with this, the development of liquid crystal compositions having negative dielectric anisotropy and compounds per se used in the compositions have also been insufficient as compared with the development of such compositions having positive dielectric anisotropy and compounds used in these compositions.

Under these circumstances, there have recently been reported wide variety of attempts, in particular, to eliminate one of disadvantages of the liquid crystal display techniques, i.e., the narrowness of the viewing angle. An example thereof is IPS mode (see, for instance, R. Kiefer et al., JAPAN DISPLAY '92, 547 (1992); M. Oh-e et al., ASIA DISPLAY '95, 577 (1995); TOKUHYO Hei 5-505247; Japanese Un-Examined Patent Publication (hereinafter referred to as "J. P. KOKAI") No. Hei 7-128647). One of the characteristic properties of IPS mode is that a comb-like electrode is arranged only on one substrate in the liquid crystal panel, while electrodes are formed on the both upper and lower substrates in the conventional liquid crystal panel. Another characteristic property of the mode is that a liquid crystal composition can be used irrespective of the dielectric anisotropy of the composition (positive or negative).

As another example of the attempts to extend the viewing angle, there may be listed a mode wherein the viewing angle is improved by making use of the vertical alignment of liquid crystal molecules (see, for instance, J.P. KOKAI No. Hei 2-176625). One of the characteristic properties of this mode is to use a liquid crystal composition having negative dielectric anisotropy.

With these points as background, it has intensively been required for the development of a liquid crystal compound having negative dielectric anisotropy and a liquid crystal composition.

Incidentally, in all of the display modes, the liquid crystal compositions used therein should have not only an appropriate dielectric anisotropy value, but also the best-controlled other characteristic properties such as values of optical anisotropy (Δn), elastic constant ratio: K₃₃/K₁₁ (K₃₃ : bend elastic constant; K₁₁ : spray elastic constant) and further they must satisfy such requirements that the temperature of the liquid crystal phase falls within an appropriate range and that they should have a low viscosity even at a low temperature.

None of conventionally known liquid crystal compounds satisfy all of these requirements when they are used alone. For this reason, a composition which can be used as a material for forming a liquid crystal material has usually been prepared by mixing several kinds to twenty or so kinds of compounds capable of exhibiting liquid crystal phases and optionally several kinds of compounds incapable of exhibiting any liquid crystal phase. Therefore, each liquid crystal compound should also satisfy such requirements that it has good miscibility with other liquid crystal compounds and that it also has good miscibility therewith in a low temperature range since the composition is also used in low temperature environments.

As has been described above, however, it has been the mainstream to use liquid crystal compositions comprising liquid crystal compounds having positive dielectric anisotropy in the conventional display modes and the development of liquid crystal compositions and compounds having negative dielectric anisotropy have still been insufficient. Therefore, the conventional liquid crystal compounds and compositions have not been able to completely cope with such diversified modes and related various characteristics. For instance, they suffer from the following various problems:
- They have negative dielectric anisotropy, but the absolute value thereof is small;
- They do not permit the reduction of the driving voltage of the display modes because of their large elastic constants;
- They cannot be used in a large amount because of poor miscibility with other ingredients for the liquid crystal compositions and the display devices using them;
- It is difficult to freely establish the optical anisotropy of these compositions or compounds;
- They have high viscosities; and
- They are physically and chemically less stable.

An example of the conventionally known liquid crystal compounds having negative dielectric anisotropy is a liquid crystal compound having a 2,3-difluorophenylene group (J.P. KOKAI No. Sho 57-114532). However, the absolute values of the negative dielectric anisotropy of these compounds are not always sufficiently large and the compounds do not have desired characteristic properties such as a low elastic constant, good miscibility with other ingredients, a high degree of freedom of optical anisotropy, a low viscosity and high physicochemical stability. For this reason, there has been desired for further improvement of these compounds and compositions.

In addition, there has not been reported any investigation to improve the foregoing characteristic properties of the compound having a 2,3-difluorophenylene group, for instance, any satisfactory attempt to increase the absolute value of the negative dielectric anisotropy thereof. In particular, there has not been proposed such an idea that a group such as tetrahydropyran-2,5-diyl group is introduced into the compound having a 2,3-difluorophenylene group.

As has been described above, the compounds of the present invention cannot easily be hit upon on the basis of the teachings or knowledges of the prior art.

Accordingly, an object of the present invention is to provide a novel liquid crystal compound which can be not only used in various display modes utilizing compounds or compositions having negative dielectric anisotropy, such as vertically aligned mode as disclosed in J.P. KOKAI No. Hei 2-176625, IPS, ECB (such as HAN or DAP), DS, GH or PC mode but also used for controlling various characteristics of liquid crystal compositions for various display modes utilizing compounds or compositions having positive dielectric anisotropy, such as TN, STN, or TN-based AM (TFT or MIM) mode, and which can solve the foregoing problems, as well as a liquid crystal composition containing the compound and a liquid crystal display device produced using the composition.

The inventors of this invention have conducted various studies to accomplish the foregoing objects and as a result, have found that the compounds as specified in the following first aspect and related embodiments of the present invention not only have negative dielectric anisotropy whose absolute values are large, but also have appropriate optical anisotropy values, small elastic constants, good miscibility with other substances, low viscosities and high physicochemical stability, that the use of the foregoing compounds permits to prescribe the formation of liquid crystal compositions whose elastic constant is small, whose dielectric and optical anisotropy values can appropriately be established, which have low viscosities and excellent physicochemical stability and, in particular, which have negative dielectric anisotropy whose absolute values are large and that an excellent liquid crystal display device can be obtained by the use of the composition and thus have completed the present invention.

According to a first aspect of the present invention, there is provided a liquid crystal compound represented by the following general Formula (2):

R¹-(A¹-X¹)ₐ₁-(A²-X²)ₐ₂-(A³-X³)ₐ₃-(A⁴-X⁴)ₐ₄-(A⁵)ₐ₅-Y¹ (2)

wherein R¹ and Y¹ each independently represents an alkyl group having 1 to 20 carbon atoms, a hydrogen atom, a halogen atom, a cyano group, a cyanate residue, an isocyano, group or an isothiocyanate residue, provided that at least one of the methylene groups present In the alkyl group, which are not adjacent to one another, may be substituted with an oxygen, sulfur or nitrogen atom, -C≡C-, a silylene group wherein at least one hydrogen atom may be substituted with an alkyl group having 1 to 10 carbon atoms, or a vinylene group, or that at least one hydrogen atom of the alkyl group may be substituted with a fluorine or chlorine atom or that these alkyl groups may be optically active ones; X¹, X², X³ and X⁴ each independently represents a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC ≡C-, or -C≡CCH=CH-; the rings A¹, A², A³, A⁴ and A⁵ each independently represents a trans-cyclohexane-1,4-diyl, cyclohexa-1-en-1,4-diyl, 1,4-phenylene, bic yclo[1.1.1]pentane-1,3-diyl, tetrahydropyran-2,5-diyl group, or a ring represented by the following general Formula (1): 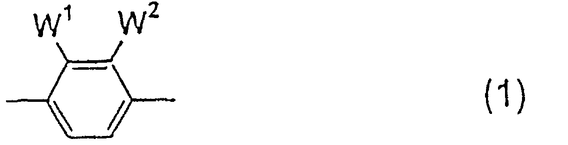 wherein W¹ and W² each independently represents a fluorine atom, provided that at least one of these rings A¹, A², A³, A⁴ and A⁵ represent a tetrahydropyran-2,5-diyl ring and at least one of them represents a ring represented by Formula (1), that the carbon atoms constituting these rings may be substituted with a nitrogen, oxygen or sulfur atom and that each hydrogen atom present on the rings may be substituted with a halogen atom or a cyano group; a¹, a², a³, a⁴ and a³ each independently represents 0 or 1, provided that they satisfy the relation: 4 ≧ a¹+a²+a³+a⁴+a⁵ ≧ 2; and each atom constituting the compound may be substituted with its isotope.

More preferably, the ring represented by Formula (1) is 2,3-difluorobenzene-1,4-diyl.

According to a second aspect of the present invention, there is provided a liquid crystal composition which comprises at least two components including at least one liquid crystal compound defined above.

According to a third aspect of the present invention, there is provided a liquid crystal composition which comprises at least one liquid crystal compound defined above as a first component and at least one compound selected from the group consisting of those represented by the following general formulas (3), (4) and (5) as a second component: 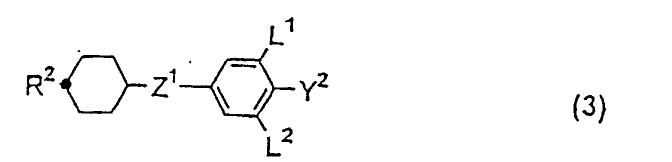 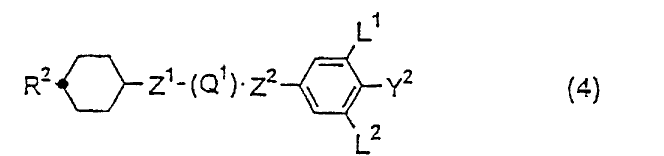 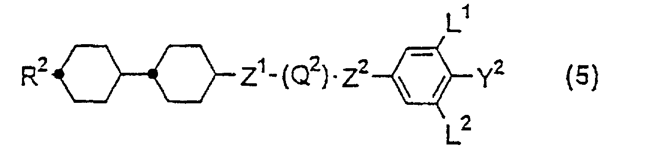 (wherein R² represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y² represents a fluorine or chlorine atom. -OCF₃, -OCF₂H, -CF₃, -CF₂H, -CFH₂, -OCF₂CF₂H or -OCF₂CFHCF₃; L¹ and L² each independently represents a hydrogen or fluorine atom; Z¹ and Z² each independently represents -(CH₂)₂-, -(CH₂)₄-, -COO-, -CF₂O-, -OCF₂-, -CH=CH- or a single bond; the ring Q¹ represents a trans-cyclohexane-1,4-diyl or 1,3-dioxane-2,5-diyl group, or a 1,4-phenylene group whose hydrogen atom may be substituted with a fluorine atom; the ring Q² represents a trans-cyclohexane-1,4-diyl group, or a 1,4-phenylene group whose hydrogen atom may be substituted with a fluorine atom; and each atom constituting the compound may be substituted with its isotope).

According to a fourth aspect of the present invention, there is provided a liquid crystal composition which comprises at least one liquid crystal compound defined above as a first component and at least one compound selected from the group consisting of those represented by the following general formulas (6) and (7) as a second component: 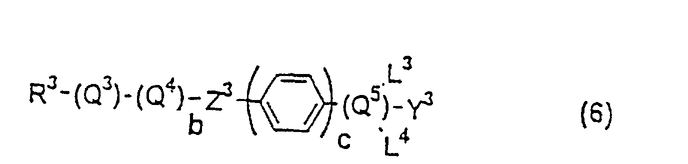 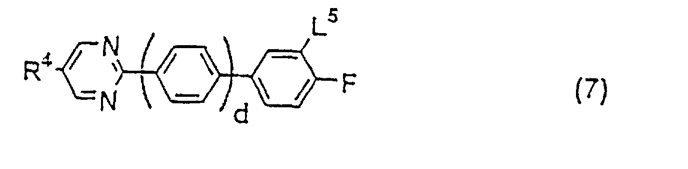 wherein R³ and R⁴ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y³ represents a cyano group or -C ≡C-CN; the ring Q³ represents a trans-cyclohexane-1,4-diyl, 1,4-phenylene, 1,3-dioxane-2,5-diyl or pyrimidine-2,5-diyl group; the ring Q⁴ represents a trans-cyclohexane-1,4-diyl or pyrimidine-2, 5-diyl group, or a 1,4-phenylene group whose hydrogen atom may be substituted with a fluorine atom; the ring Q⁵ represents a transcyclohexane-1,4-diyl or 1,4-phenylene group; Z³ represents -(CH₂)₂-, -COO- or a single bond; L³, L⁴ and L⁵ each independently represents a hydrogen or fluorine atom; b, c and d each independently represents 0 or 1; and each atom constituting the compound may be substituted with its isotope.

According to a fifth aspect of the present invention, there is provided a liquid crystal composition which comprises at least one liquid crystal compound defined above as a first component; at least one compound selected from the group consisting of those represented by the foregoing general formulas (3), (4) and (5) as a second component; and at least one compound selected from the group consisting of those represented by the following general formulas (8), (9) and (10) as a third component:

R⁵·(Q⁶)-Z⁴·(Q⁷)-Z⁵―R⁶ (8)

R⁵·(Q⁶)-Z⁴·(Q⁷)-Z⁵-(Q⁸)·R⁶ (9)

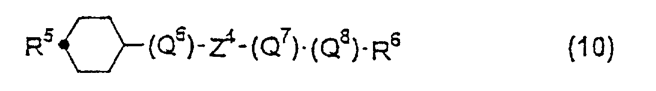

wherein R⁵ and R⁶ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁶, Q⁷ and Q⁸ each independently represents a trans-cyclohexane-1,4-diyl or pyrimidine-2,5-diyl group, or a 1,4-phenylene group whose hydrogen atom may be substituted with a fluorine atom; Z⁴ and Z⁵ each independently represents -C≡C-, -COO-, -(CH₂)₂-, -CH=CH- or a single bond; and each atom constituting the compound may be substituted with its isotope.

According to a sixth aspect of the present invention, there is provided a liquid crystal composition which comprises at least one liquid crystal compound defined above as a first component and at least one compound selected from the group consisting of those represented by the following general formulas (11), (12) and (13) as a second component: 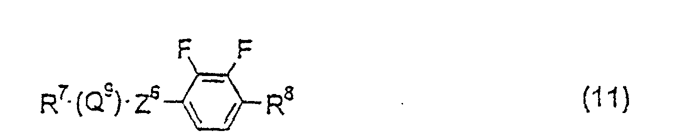 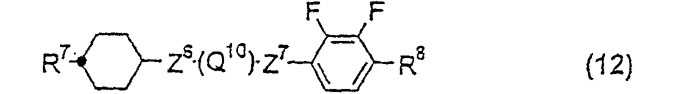 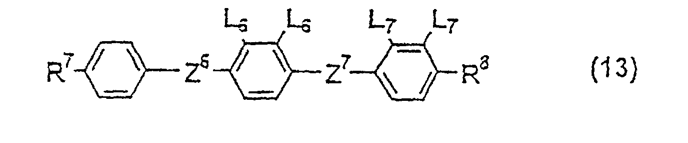 wherein R⁷ and R⁸ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁹ and Q¹⁰ each independently represents a trans-cyclohexane-1,4-diyl or 1,4-phenylene group; L⁶ and L⁷ each independently represents a hydrogen or fluorine atom, provided that L⁶ and L⁷ do not simultaneously represent a hydrogen atom; Z⁶ and Z⁷ each independently represents -(CH₂)₂-, -COO- or a single bond; and each atom constituting the compound may be substituted with its isotope.

According to a seventh aspect of the present invention, there is provided a liquid crystal composition which comprises at least one liquid crystal compound defined above as a first component; at least one compound selected from the group consisting of those represented by the foregoing general formulas (8), (9) and (10) as a second component; and at least one compound selected from the group consisting of those represented by the foregoing general formulas (11), (12) and (13) as a third component.

According to an eighth aspect of the present invention, there is provided a liquid crystal composition which comprises at least one liquid crystal compound defined above as a first component; at least one compound selected from the group consisting of those represented by the foregoing general formulas (6) and (7) as a second component; and at least one compound selected from the group consisting of those represented by the foregoing general formulas (8), (9) and (10) as a third component.

According to a ninth aspect of the present invention, there is provided a liquid crystal composition which comprises at least one liquid crystal compound defined above as a first component; at least one compound selected from the group consisting of those represented by the foregoing general formulas (3), (4) and (5) as a second component; at least one compound selected from the group consisting of those represented by the foregoing general formulas (6) and (7) as a third component; and at least one compound selected from the group consisting of those represented by the foregoing general formulas (8). (9) and (10) as a fourth component.

According to a tenth aspect of the present invention, there is provided a liquid crystal composition which comprises at least one optically active compound in addition to one of the liquid crystal compositions according to the second to ninth aspects of the present invention.

According to an eleventh aspect of the present invention, there is provided a liquid crystal display device constituted by the use of one of the liquid crystal compositions according to the second to tenth aspects of the present invention.

The sixth and seventh aspects of the present invention relate to N-type (having negative Δε) liquid crystal compositions. The N-type liquid crystal composition can be driven according to a variety of display modes such as vertically aligned and IPS modes as disclosed in J.P. KOKAI No. Hei 2-176625.

Moreover, the present inventions according to the third, fourth, fifth, eighth and ninth aspects of the present invention relate to P-type (having positive Δ ε) liquid crystal compositions, but an N-type compound may also be used as a component for such P-type liquid crystal compositions. This permits not only arbitrary establishment of the dielectric anisotropy value of the liquid crystal composition depending on any particular use, but also the control of other characteristic properties of the composition such as the optical anisotropy value and the elastic constant.

### Description of the Preferred Embodiments

Preferred embodiments of the compounds of the present invention, represented by Formula (2) will be described below.

The substituents R¹ and Y¹ of the compound of Formula (2) each independently represents an alkyl group having 1 to 20 carbon atoms, a hydrogen or halogen atom, a cyano or isocyano group, or a cyanate or isothiocyanate residue, provided that at least one of the methylene groups present in the alkyl group, which are not adjacent to each other, may be substituted with an oxygen, sulfur or nitrogen atom, a group -C≡C-, a silylene group in which at least one hydrogen atom may be substituted with an alkyl group having 1 to 10 carbon atoms, or a vinylene group, that at least one of the hydrogen atoms present in the alkyl group may be substituted with a fluorine or chlorine atom or that these alkyl groups may be optically active ones.

Specific examples of the alkyl group represented by R¹ and Y¹ in Formula (2) include saturated alkyl groups simply consisting of carbon and hydrogen atoms, haloalkyl groups, alkoxy or haloalkoxy groups, alkenyl or haloalkenyl groups, alkenyloxy or haloalkenyloxy groups, alkynyl or haloalkynyl groups, alkynyloxy or haloalkynyloxy groups, alkoxyalkyl or haloalkoxyalkyl groups, alkoxyalkoxy or haloalkoxyalkoxy groups, alkanoyl or haloalkanoyl groups, alkanoyloxy or haloalkanoyloxy groups, and alkoxycarbonyl or haloalkoxycarbonyl groups.

Specific examples of R¹ and Y¹ are groups or atoms described below:
-CH₃, -C₂H₅, -C₃H₇, -C₄H₉, -C₅H₁₁, -C₆H₁₃, -C₇H₁₅, -C₈H₁₇, -C₉H₁₉, -C₁₀H₂₁, -CFH₂, -CF₂H, -CF₃, -CH₂CH₂F, -CH₂CHF₂, -CH₂CF₃, -CF₂CH₃, -CF₂CF₂H, -CHFCF₃, -CF₂CF₃, -(CH₂)₂CH₂F, -(CH₂)₂CHF₂, -(CH₂)₂CF₃, -CH₂CF₂CH₃, -CH₂CF₂CF₃, -CF₂C₂H₅, -C₃F₇, -CF₂CFHCF₃, -(CH₂)₃CH₂F, -(CH₂)₄CH₂F, -(CH₂)₅CH₂F, -CF₂C₃H₇, -CH₂CF₂C₂H₅, -(CH₂)₂CF₂CH₃, -CF₂C₄H₉, -CH₂CF₂CH₃, -(CH₂)₂CF₂C₂H₅, -(CH₂)₃CF₂CH₃, -OCH₃, -OC₂H₅, -OC₃H₇, -OC₄H₉, -OC₅H₁₁, -OC₆H₁₃, -OC₇H₁₅, -OC₈H₁₇, -OC₉H₁₉, -OC₁₀H₂₁, -OCFH₂, -OCF₂H, -OCF₃, -OCH₂CH₂F, -OCH₂CHF₂, -OCH₂CF₃, -OCF₂CH₃, -OCF₂CF₂H, -OCHFCF₃, -OCF₂CF₃, -O(CH₂)₂CH₂F, -O(CH₂)₂CHF₂, -O(CH₂)₂CF₃, -OCH₂CF₂CH₃, -OCH₂CF₂CF₃, -OCF₂C₂H₅, -OC₃F₇, -OCF₂CFHCF₃, -O(CH₂)₃CH₂F, -O(CH₂)₄CH₂F, -O(CH₂)₅CH₂F, -OCF₂C₃H₇, -OCH₂CF₂C₂H₅, -O(CH₂)₂CF₂CH₃, -OCF₂C₄H₉, -OCH₂CF₂CH₃, -O(CH₂)₂CF₂C₂H₅, -O(CH₂)₃CF₂CH₃, -CH=CH₂, -CH=CHCH₃, -CH=CHC₂H₅, -CH=CHC₃H₇, -CH=CHC₄H₉, -CH₂CH=CHCH₃, -CH₂CH=CHC₂H₅, -CH₂CH=CHC₃H₇, -CH₂CH=CHC₄H₉, -(CH₂)₂CH=CHCH₃, -(CH₂)₂CH=CHC₂H₅, -(CH₂)₂CH=CHC₃H₇, -(CH₂)₂CH=CHC₄H₉, -CH₂CH=CH₂, -(CH₂)₂CH=CH₂, -(CH₂)₃CH=CH₂, -(CH₂)₄CH=CH₂, -(CH₂)₅CH=CH₂, -CH=CHCH=CH₂, -CH=CHCH₂CH=CH₂, -CH=CH(CH₂)₂CH=CH₂, -CH=CH(CH₂)₃CH=CH₂, -CH₂CH=CH(CH₂)₂CH=CH₂, -(CH₂)₂CH=CH(CH₂)₂CH=CH₂, -CF=CF₂, -CH=CF₂, -CH=CHF, -CH₂CF=CF₂, -CH₂CH=CHF, -(CH₂)₂CH=CF₂, -(CH₂)₂CH=CHF, -(CH₂)₃CH=CF₂, -(CH₂)₃CH=CHF, -(CH₂)₄CH=CF₂, -(CH₂)₄CH=CHF, -(CH₂)₅CH=CF₂, -(CH₂)₅CH=CHF, -CH=CHCH₂CH₂F, -CH=CHCH₂CH₂Cl, -CH=CHCH₂CHF₂, -CH=CHCH₂CF₃, -CH=CH(CH₂)₂CH₂F, -CH=CH(CH₂)₂CHF₂, -CH=CH(CH₂)₂CF₃, -CH=CH(CH₂)₃CH₂F, -CH=CH(CH₂)₃CHF₂, -CH=CH(CH₂)₃CF₃, -CH=CH(CH₂)₄CH₂F, -CH=CH(CH₂)₄CHF₂, -CH=CH(CH₂)₄CF₃, -CH₂CH=CH(CH₂)₂CH₂F, -CH₂CH=CHCH₂CH₂F, -(CH₂)₂CH=CH(CH₂)₂CH₂F, -(CH₂)₂CH=CHCH₂CH₂F, -CH=CHCH₂CH=CF₂, -CH=CH(CH₂)₂CH=CF₂, -CH=CH(CH₂)₂CF=CF₂, -CH=CH(CH₂)₃CHFCH₃, -OCH₂CH=CHCH₃, -OCH₂CH=CHC₂H₅, -OCH₂CH=CHC₃H₇, -OCH₂CH=CHC₄H₉, -O(CH₂)₂CH=CHCH₃, -O(CH₂)₂CH=CHC₂H₅, -O(CH₂)₂CH=CHC₃H₇, -O(CH₂)₂CH=CHC₄H₉, -OCH₂CH=CH₂, -O(CH₂)₂CH=CH₂, -O(CH₂)₃CH=CH₂, -O(CH₂)₄CH=CH₂, -O(CH₂)₅CH=CH₂, -OCH₂CH=CH(CH₂)₂CH=CH₂, -O(CH₂)₂CH=CH(CH₂)₂CH=CH₂, -OCH₂CF=CF₂, -OCH₂CH=CF₂, -OCH₂CF=CHF, -O(CH₂)₂CH=CF₂, -O(CH₂)₂CH=CHF, -O(CH₂)₃CH=CF₂, -O(CH₂)₃CH=CHF, -O(CH₂)₄CH=CF₂, -O(CH₂)₄CH=CHF, -O(CH₂)₅CH=CF₂, -O(CH₂)₅CH=CHF, -OCH₂CH=CHCH₂CH₂F, -O(CH₂)₂CH=CH(CH₂)₂CH₂F, -O(CH₂)₂CH=CHCH₂CH₂F, -C ≡CH, -C≡CCH₃, -C≡CC₂H₅, -C≡CC₃H₇, -C≡CC₄H₉, -CH₂C≡CH, -CH₂C≡CCH₃. -CH₂C≡CC₂H₅, -CH₂C≡CC₃H₇, -CH₂C≡CC₄H₉, -(CH₂)₂C≡CH, -(CH₂)₂C≡CCH₃, -(CH₂)₂C≡CC₂H₅, -(CH₂)₂C ≡CC₃H₇, -(CH₂)₂C≡CC₄H₉, -(CH₂)₃C≡CH, -(CH₂)₃C≡CCH₃, -(CH₂)₃C≡CC₂H₅, -(CH₂)₃C≡CC₃H₇, -(CH₂)₃C≡CC₄H₉, -C≡CCF₃, -C≡CC₂F₅, -C≡CC₃F₇, -CH₂C≡CCF₃, -(CH₂)₂C≡CCF₃, -OCH₂C≡CH, -OCH₂C≡CCH₃, -OCH₂C≡CC₂H₅, -OCH₂C≡CC₃H₇, -OCH₂C≡CC₄H₉, -O(CH₂)₂C≡CH, -O(CH₂)₂C≡CCH₃, -O(CH₂)₂C≡CC₂H₅, -O(CH₂)₂C≡CC₃H₇, -O(CH₂)₂C≡CC₄H₉, -O(CH₂)₃C≡CH, -O(CH₂)₃C≡CCH₃, -O(CH₂)₃C≡CC₂H₅, -O(CH₂)₃C≡CC₃H₇, -O(CH₂)₃C≡CC₄H₉, -OCH₂C≡CCF₃, -O(CH₂)₂C≡CCF₃, -CH₂OCH₃, -CH₂OC₂H₅, -CH₂OC₃H₇, -CH₂OC₄H₉, -CH₂OC₅H₁₁, -(CH₂)₂OCH₃, -(CH₂)₂OC₂H₅, -(CH₂)₂OC₃H₇, -(CH₂)₂OC₄H₉, -(CH₂)₂OC₅H₁₁, -(CH₂)₃OCH₃, -(CH₂)₃OC₂H₅, -(CH₂)₃OC₃H₇, -(CH₂)₃OC₄H₉, -(CH₂)₃OC₅H₁₁, -(CH₂)₄OCH₃, -CF₂OCH₃, -CF₂OC₂H₅, -CF₂OC₃H₇, -CF₂OC₄H₉, -CF₂OC₅H₁₁, -CH₂OCF₃, -CH₂OC₂F₅, -OCH₂OCH₃, -OCH₂OC₂H₅, -OCH₂OC₃H₇, -OCH₂OC₄H₉, -OCH₂OC₅H₁₁, -O(CH₂)₂OCH₃, -O(CH₂)₂OC₂H₅, -O(CH₂)₂OC₃H₇, -O(CH₂)₂OC₄H₉, -O(CH₂)₂OC₅H₁₁, -O(CH₂)₃OCH₃, -O(CH₂)₃OC₂H₅, -O(CH₂)₃OC₃H₇, -O(CH₂)₃OC₄H₉, -O(CH₂)₃OC₅H₁₁, -O(CH₂)₄OCH₃, -OCH₂OCF₃, -OCH₂OC₂F₅, -COCF₃, -COCHF₂, -COC₂F₅, -COC₃F₇, -COCH₂CF₃, -OCOCH₃, -OCOC₂H₅, -OCOC₃H₇, -OCOC₅H₁₁, -OCOC₇H₁₅, -OCOCF₃, -OCOCHF₂, -OCOC₂F₅, -OCOC₃F₇, -OCOCH₂CF₃, -COOCH₃, -COOC₂H₅, -COOC₃H₇, -COOC₄H₉, -COOC₅H₁₁, -COOCH(CH₃)C₈H₁₃, -COOCH₂F, -COOCH₂CF₃, -COO(CH₂)₂CF₃, -COO(CH₂)₃CF₃, -COO(CH₂)₄CH₂F, hydrogen or halogen atoms, cyano or isocyano groups, and cyanate or isothiocyanate residues.

If paying attention, in particular, to the physicochemical stability of the compound of Formula (2), R¹ and Y¹ are preferably saturated alkyl groups simply consisting of carbon and hydrogen atoms, haloalkyl groups, alkoxy or haloalkoxy groups, alkoxyalkyl or haloalkoxyalkyl groups, alkoxyalkoxy or haloalkoxyalkoxy groups, alkanoyl or haloalkanoyl groups, alkanoyloxy or haloalkanoyloxy groups, alkoxycarbonyl or haloalkoxycarbonyl groups, hydrogen or halogen atoms, cyano or isocyano groups, and cyanate residues.

If it is desired to further increase the absolute value of the negative dielectric anisotropy of the compound of Formula (2), R¹ and Y¹ are preferably substituents having a low polarity such as saturated alkyl groups simply consisting of carbon and hydrogen atoms, alkoxy groups, alkenyl groups, alkenyloxy groups, alkynyl groups, alkynyloxy groups, alkoxyalkyl groups, alkoxyalkoxy groups, alkanoyl groups, alkanoyloxy groups, alkoxycarbonyl groups, or hydrogen atom.

The substituents X¹, X², X³ and X⁴ present on the compound of Formula (2) are as follows: a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC ≡C-, and -C≡CCH=CH-.

If paying attention, in particular, to the physicochemical stability of the compound of Formula (2), preferred substituents X¹, X², X³ and X4 are a single bond, -(CH₂)₂-, -COO-, -OCO-, -CH₂O-, -OCH₂-, -(CH₂)₄-, -(CH₂)₃O-, -O(CH₂)₃-, -CF₂O-, -OCF₂-, -CH₂CF₂-, -CF₂CH₂-, -(CF₂)₂-, -(CF₂)₄-, -(CH₂)₂COO-, and -OCO(CH₂)₂-.

At least one of the rings A¹, A², A³, A⁴ and A⁵ of the compound represented by Formula (2) is a tetrahydropyran-2,5-diyl ring and at least one of them is a ring represented by Formula (1).

Preferred rings other than the foregoing are, for instance, bicyclo[1.1.1]pentane-1,3-diyl, trans-cyclohexane-1,4-diyl, cyclohexa-1-en-1,4-diyl and 1,4-phenylene, provided that the carbon atoms constituting these rings may be substituted with a nitrogen, oxygen or sulfur atom and that the hydrogen atoms present on the rings may likewise be substituted with a halogen atom or a cyano group.

Specific examples thereof include 1-cyano-trans-cyclohexane-1,4-diyl, 2,2-difluoro-trans-cyclohexane-1,4-diyl, 2-fluorocyclohexa-1-ene-1,4-diyl, and 2,2-dichlorobicyclo[1.1.1]pentane-1,3-diyl. 1. Examples of the rings whose hydrogen atoms present thereon may be substituted with a fluorine or chlorine atom or a cyano group are as follows:

Bicyclo[1.1.1]pentane-1,3-diyl, trans-cyclohexane-1,4-diyl, cyclohexa-1-ene-1,4-diyl, 1,4-phenylene, 1,3-dioxane-2,5-diyl, pyrimidine-2,5-diyl, pyridine-2,5-diyl, 1, pyrazine-2,5-diyl, pyridazine-3,6-diyl, 1,3-dithian-2,5-diyl, 1,3-oxathian-2,5-diyl, 2,3-difluorobenzene-1,4-diyl and bicyclo[2.2.2]octane-1,4-diyl.

If paying attention, in particular, to the physicochemical stability of the compound of Formula (2), preferred rings A¹, A², A³, A⁴ and A⁵ of the compound include tetrahydropyran-2,5-diyl rings and those represented by Formula (1) (such as 2,3-difluorobenzene-1,4-diyl), but preferred rings A¹, A², A³, A⁴ and A⁵ other than the foregoing are as follows:

1-cyano-trans-cyclohexane-1,4-diyl, 2,2-difluoro-transcyclohexane-1,4-diyl, 2-fluorocyclohexa-1-ene-1,4-diyl, and 2,2-dichlorobicyclo[1.1.1]pentane-1,3-diyl.

Examples of the rings whose hydrogen atoms present thereon may be substituted with a fluorine or chlorine atom or a cyano group are bicyclo[1.1.1]pentane-1,3-diyl, trans-cyclohexane-1,4-diyl, cyclohexa-1-ene-1,4-diyl, 1,4-phenylene, 1,3-dioxane-2,5-diyl, pyrimidine-2,5-diyl, pyridine-2,5-diyl, pyridazine-3,6-diyl, 1,3-dithian-2,5-diyl, 2,3-difluorobenzene-1,4-diyl, and 1,3-oxathian-2,5-diyl.

If it is desired to further increase the absolute value of the negative dielectric anisotropy of the compound of Formula (2), preferred rings A¹, A², A³, A⁴ and A⁵ are tetrahydropyran-2,5-diyl rings and those represented by Formula (1) (such as 2,3-difluorobenzene-1,4-diyl), but preferred rings A¹, A², A³, A⁴ and A⁵ other than those listed above are 1-cyano-trans-cyclohexane-1,4-diyl, 2,2-difluoro-trans-cyclohexane-1,4-diyl, 2-fluorocyclohexa-1-ene-1,4-diyl, and 2,2-dichlorobicyclo[1.1.1]pentane-1,3-diyl.

Examples of the rings whose hydrogen atoms present thereon may be substituted with a fluorine or chlorine atom or a cyano group include trans-cyclohexane-1,4-diyl, cyclohexa-1-ene-1,4-diyl, and pyridazine-3,6-diyl.

The compounds according to the first aspect of the present invention and preferred embodiments thereof have negative dielectric anisotropy values whose absolute values are large. Therefore, a liquid crystal composition having a negative dielectric anisotropy value can be prepared by mainly using the foregoing compounds. This liquid crystal composition is useful in the preparation of devices which make use of a liquid crystal composition having negative dielectric anisotropy, including, for instance, vertically aligned modes and IPS's as disclosed in J.P. KOKAI No. Hei 2-176625. Moreover, the dielectric anisotropy value can appropriately be established by the use of a mixture of the compound of the present invention with other liquid crystal compounds or compositions to thus expand the area of applications of these other liquid crystal compounds or compositions.

The compounds according to the first aspect of the present invention and preferred embodiments thereof have small elastic constants and the temperature-dependency of the constants is also low. Therefore, the use of the compounds of the present invention would permit the preparation of novel liquid crystal compositions having a low driving voltage.

The compounds according to the first aspect of the present invention and preferred embodiments thereof exhibit good miscibility with other liquid crystal compounds. For this reason, if the compounds of the present invention are incorporated into liquid crystal compositions, any problem such as separation of compounds rarely arises. In addition, a large amount of the compound of the present invention can be incorporated into a liquid crystal composition and accordingly, the excellent characteristics of the compound of the present invention can intensively be reflected in the resulting liquid crystal composition.

The compounds according to the first aspect of the present invention and preferred embodiments thereof permit appropriate design of the structure of the compounds such that they have a desired level of the optical anisotropy value. In other words, the compound should be designed so as to have rings including a large number of sites having resonance structures such as aromatic rings, if it is necessary to use a compound having a particularly high optical anisotropy value. On the other hand, if it is necessary to use a compound having a low optical anisotropy value, the compound should be designed so as to have a ring including a large number of sites free of any resonance structure such as trans-cyclohexane-1,4-diyl ring.

If describing this more fully, the compound of Formula (2) according to the present invention may have a desired level of the optical anisotropy value by appropriately selecting the substituents R¹, Y¹, X¹, X², X³, X⁴, A¹, A², A³, A⁴, A⁵, a¹, a², a³, a⁴ and a⁵. More specifically, the optical anisotropy value may be controlled by appropriately selecting the rings A¹, A², A³, A⁴ and A⁵. In other words, the selection of a ring or rings each including a large number of sites having resonance structures such as aromatic rings permits the preparation of a compound having particularly high optical anisotropy, while the selection of a ring or rings each including a large number of sites free of any resonance structure such as transcyclohexane-1,4-diyl ring permits the preparation of a compound having low optical anisotropy.

The degree of freedom in the design of a liquid crystal panel would widely be expanded by arbitrarily selecting the optical anisotropy value.

All of the compounds according to the first aspect of the present invention and preferred embodiments thereof have low viscosities and therefore, they do not undesirably increase the overall viscosity of the resulting liquid crystal composition even if a large amount of the compound is incorporated into the composition. In addition, the compound of the present invention exhibits very low temperature-dependency of the viscosity, in particular, at a low temperature. The use of the liquid crystal compound having such an excellent viscosity permits the preparation of a liquid crystal composition having high speed responsibility.

All of the compounds according to the first aspect of the present invention and preferred embodiments thereof are physicochemically stable and accordingly, the liquid crystal compositions prepared using them have high specific resistance and a high voltage-holding ratio. In other words, the compounds are highly stable against external factors such as ultraviolet rays and heat and have physicochemical stability sufficient for use as ingredients of practically used liquid crystal compositions.

The phase transition temperature of the compounds according to the first aspect of the present invention and preferred embodiments thereof can be controlled by appropriately selecting the number of rings attached to each compound. In other words, it would be sufficient to select a compound carrying not less than 4 rings when it is desired for the achievement of a particularly high clearing point, a compound having 3 rings, when it is desired for the achievement of a medium clearing point, and a compound having 2 rings when it is desired for the achievement of a particularly low clearing point.

If describing this more fully, the phase transition points of the compounds of Formula (2) according to the present invention can be controlled by appropriately selecting the values of a¹, a², a³, a⁴ and a⁵. More specifically, it would be sufficient to select a compound having 4 rings and a¹+a²+a³+a⁴+a⁵ = 4 when it is desired for the achievement of a particularly high clearing point; a compound having 3 rings and a¹+a²+a³+a⁴+a⁵=3 when it is desired for the achievement of a medium clearing point; and a compound having 2 rings and a¹+a²+a³+a⁴+a⁵=2 when it is desired for the achievement of a particularly low clearing point.

As has been discussed above, the compounds of the first aspect of the present invention and the preferred embodiments thereof have various characteristic properties favorable for use as electro-optical display materials. The use of the compounds of the present invention permits the preparation of novel liquid crystal compositions having good characteristics. In addition, the present invention also permits the production of liquid crystal compounds having desired properties by appropriately designing the structure thereof depending on each particular use as well as liquid crystal composition and liquid crystal display devices using the compounds.

For instance, the compound of the present invention is characterized by having a large negative dielectric anisotropy value, but the compound can not only be used in a liquid crystal composition having a negative dielectric anisotropy value, but also can be used to control the dielectric anisotropy value and other characteristics of a composition having a positive dielectric anisotropy value by the addition of the former to the latter.

If describing this more fully, the compound of the present invention and the liquid crystal composition including the same can be used in various display modes which make use of compounds or compositions having negative dielectric anisotropy values (such as the vertically aligned modes as disclosed in J.P. KOKAI No. Hei 2-176625, IPS, ECB (for instance, HAN or DAP), DS, GH or PC mode), in particular, the vertically aligned mode as disclosed in J.P. KOKAI No. Hei 2-176625 and IPS. In addition to the foregoing, the compound and composition of the present invention can also be used for the improvement or control of various characteristics (for instance, dielectric anisotropy, elastic constant, optical anisotropy, viscosity, and/or physicochemical stability) of liquid crystal compositions used for various display modes (such as TN, STN, or TN-based AM (e.g., TFT or MIM) mode) which make use of compounds or compositions having positive dielectric anisotropy values.

The liquid crystal composition of the present invention will now be detailed below. The liquid crystal composition of the present invention preferably comprises 0.1 to 99.9% by weight of at least one compound as defined in the first aspect of the present invention and the preferred embodiments thereof, based on the total weight of the composition, in order to impart excellent characteristic properties to the composition.

More specifically, the liquid crystal composition of the present invention comprises a compound selected from the group consisting of those represented by Formulas (3) to (13) in an appropriate amount depending on each particular purpose, in addition to the compound of the present invention as a first component.

The following are examples of preferred compounds represented by Formulas (3) to (5) which can be used in the liquid crystal composition of the present invention (in these compounds, R² and Y² are the same as those defined above):

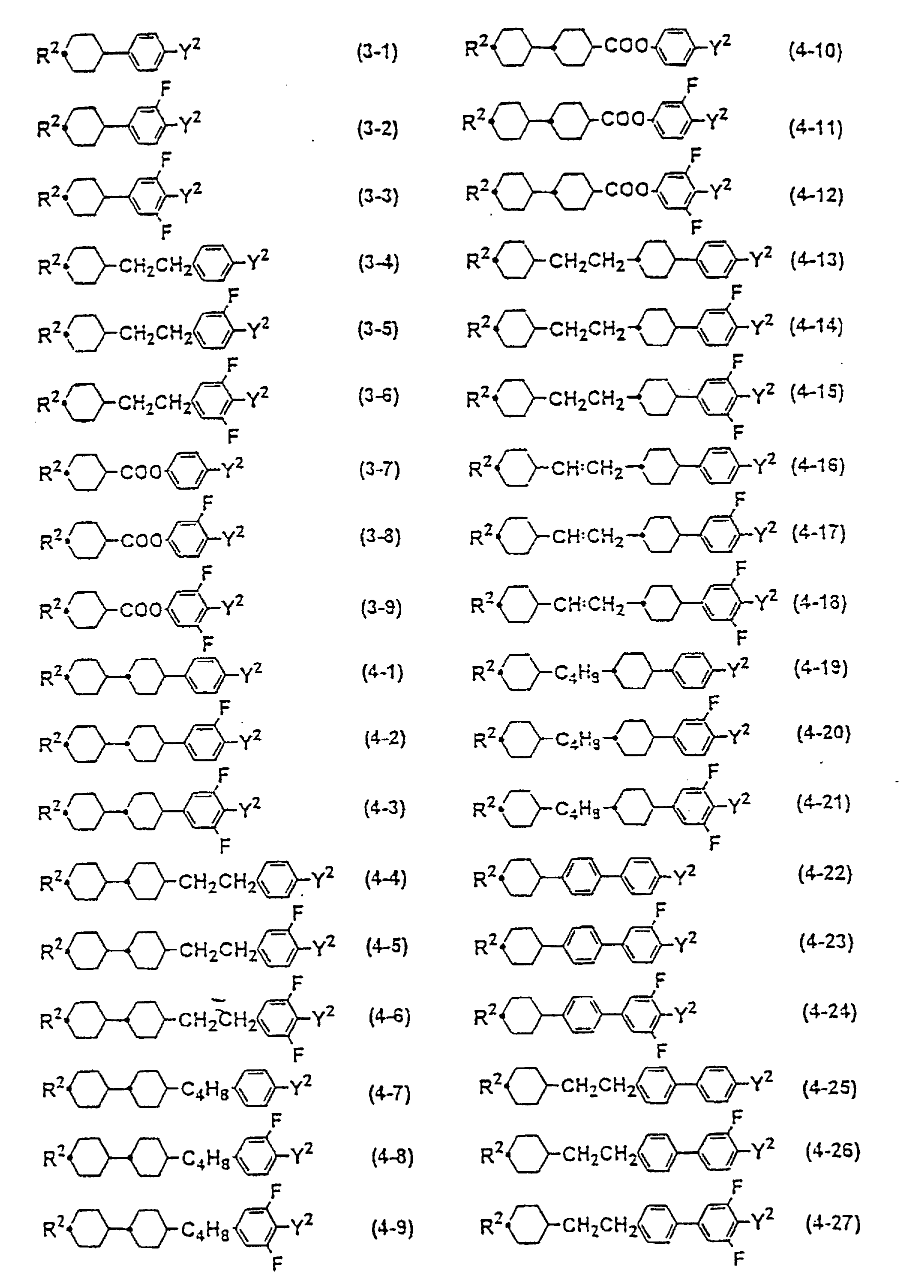

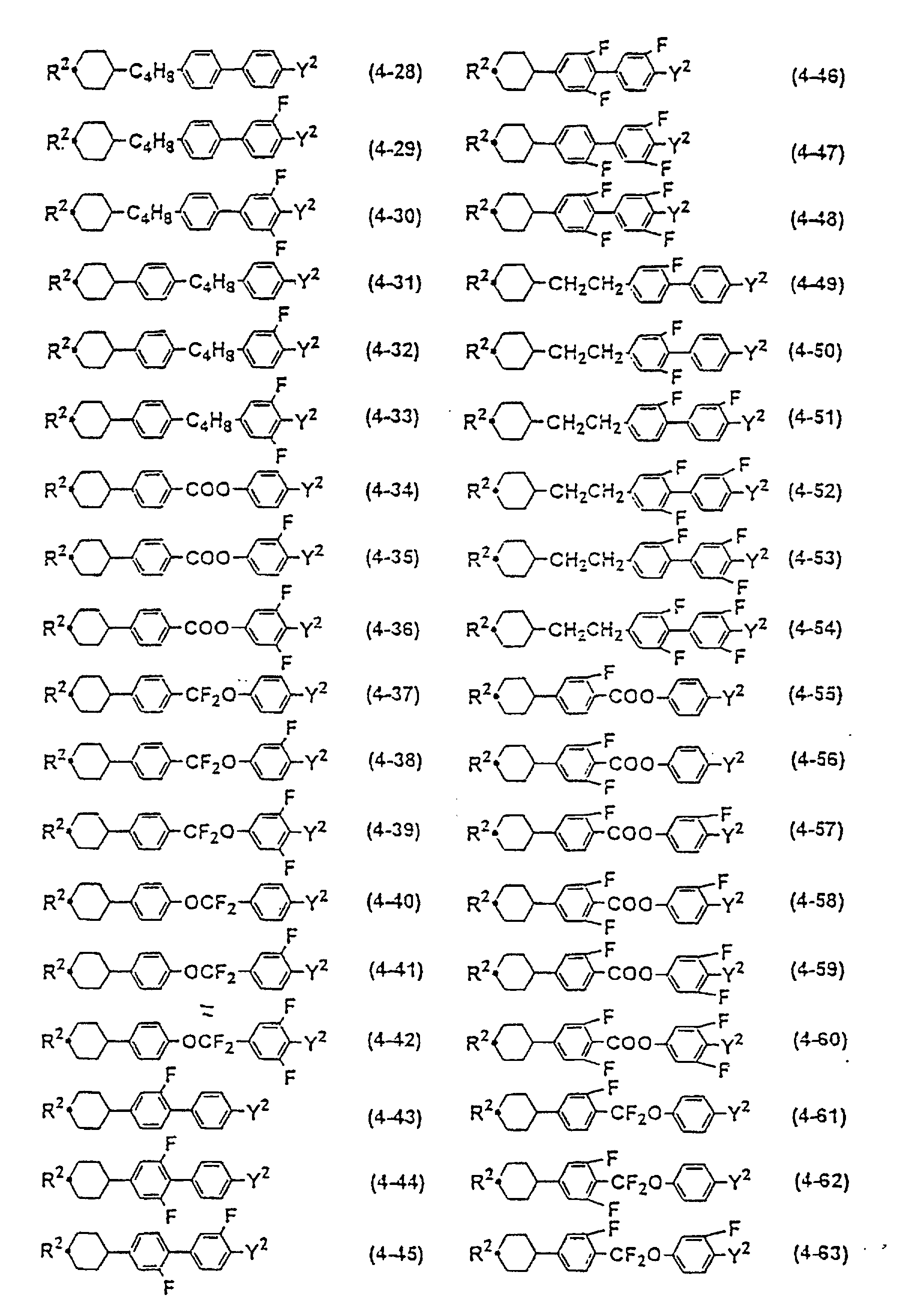

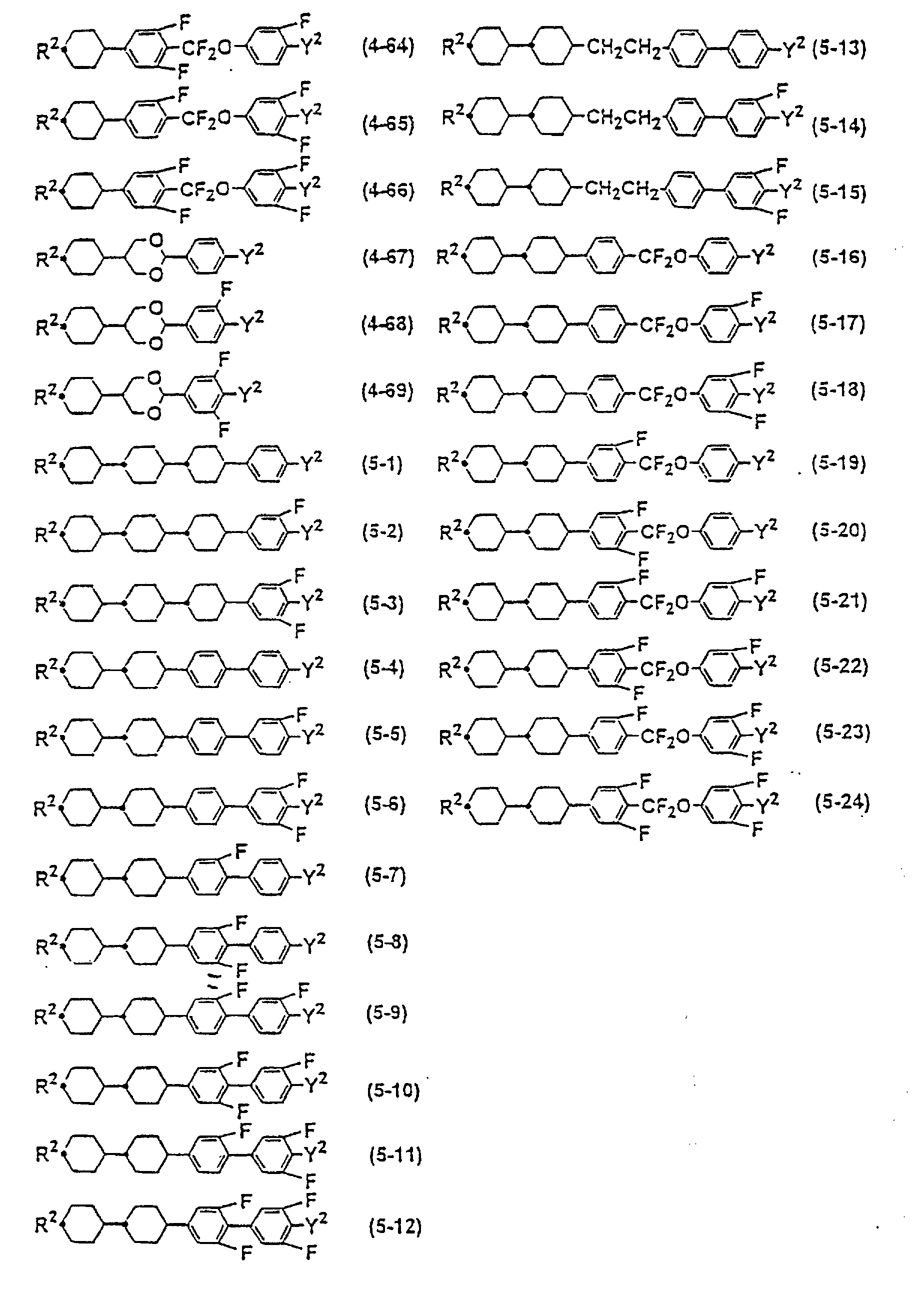

The compounds represented by Formulas (3) to (5) have positive dielectric anisotropy values, are quite excellent in thermal and chemical stability and are particularly preferably used when preparing liquid crystal compositions used in TFT's which require high reliability such as a high voltage-holding ratio or a high specific resistance.

The amount of the compounds of Formulas (3) to (5) used when preparing a liquid crystal composition used in TFT's ranges from 0.1 to 99.9% by weight, preferably 10 to 97% by weight and more preferably 40 to 95% by weight based on the total weight of the composition. In addition, the liquid crystal composition may further comprise the compounds of Formulas (8) to (10) in order to control the viscosity of the composition.

The compounds represented by Formulas (3) to (5) can likewise be used when preparing a liquid crystal composition for STN's or TN's. In this case, they are preferably used in an amount of not more than 50% by weight based on the total weight of the composition.

The following are examples of preferred compounds represented bY Formulas (6) to (7) which can be used in the liquid crystal composition of the present invention (in these compounds, R³, Y³ and R⁴ are the same as those defined above):

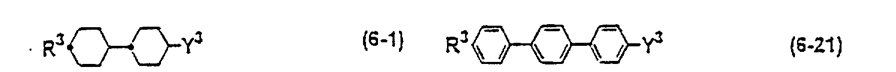

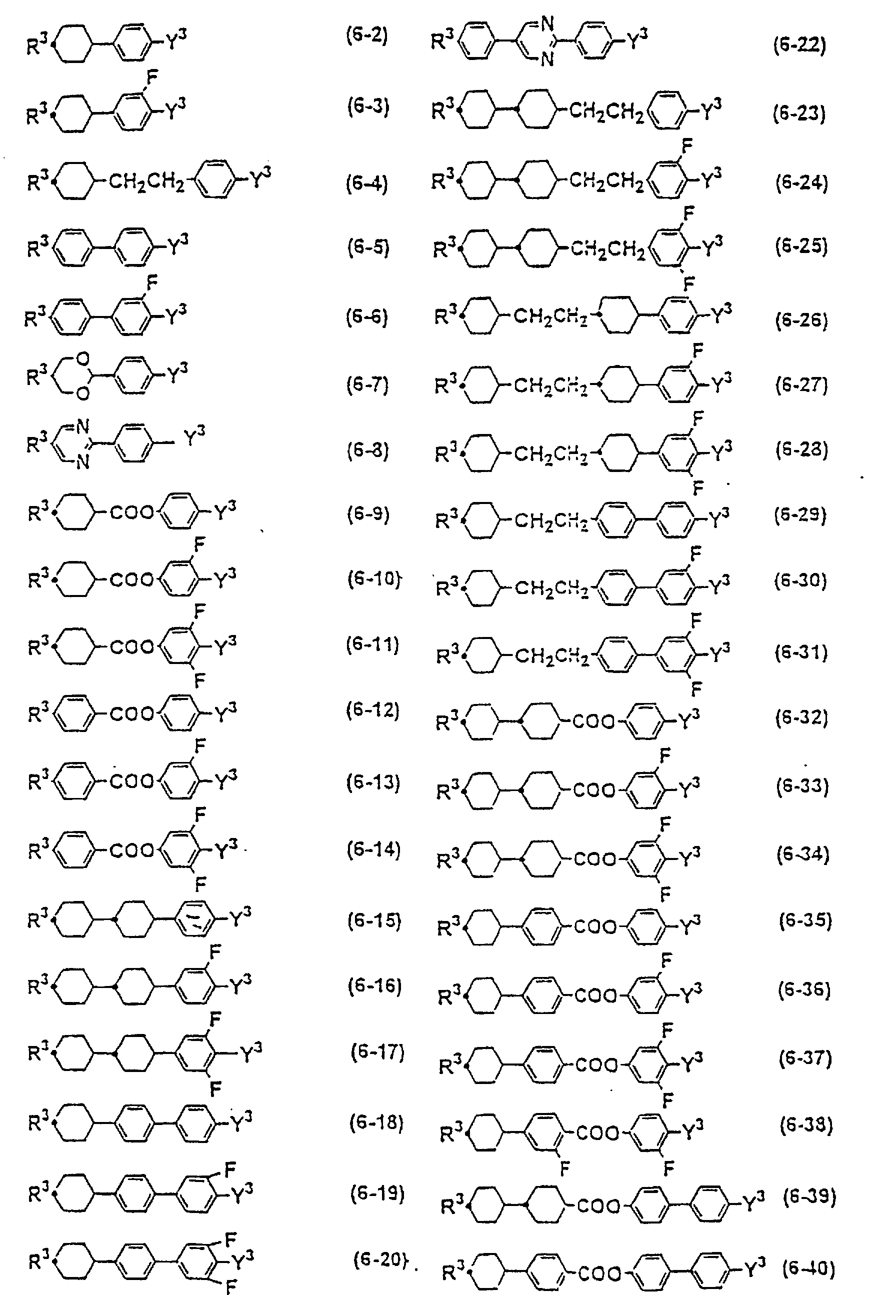

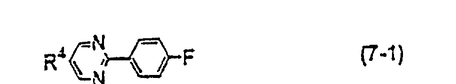

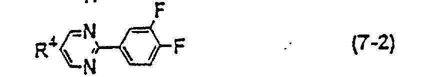

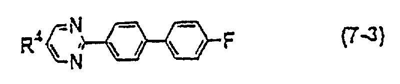

The compounds represented by Formulas (6) to (7) have large and positive dielectric anisotropy values and are used, in particular, for the purpose of reducing the threshold voltage of the resulting liquid crystal composition. Moreover, they can be used for controlling the optical anisotropy value and for expanding the nematic range, for instance, increasing the clearing point. Further they are also used for the improvement in the steepness of the V-T curve of the liquid crystal composition for STN's or TN's.

The compounds represented by Formulas (6) to (7) are preferred, in particular, when preparing the liquid crystal composition for STN's or TN's.

If the amount of the compound of Formula (6) or (7) used increases, the threshold voltage of the resulting liquid crystal composition is reduced and the viscosity thereof increases. Therefore, it is advantageous to use the compounds in a large amount since the resulting mode can be operated at a low voltage, so far as the composition satisfies the requirement for viscosity. If preparing a liquid crystal composition for STN's or TN's, the amount of the compound of Formula (6) or (7) ranges from 0.1 to 99.9% by weight, preferably 10 to 97% by weight and more preferably 40 to 95% by weight based on the total weight of the composition.

The following are examples of preferred compounds represented by Formulas (8) to (10) which can be used in the liquid crystal composition of the present invention (in these compounds, R⁵ and R⁶ are the same as those defined above):

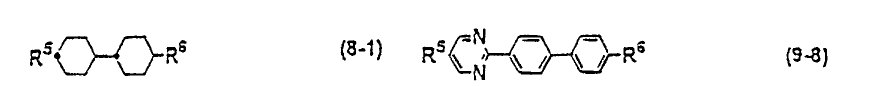

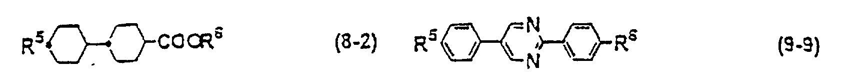

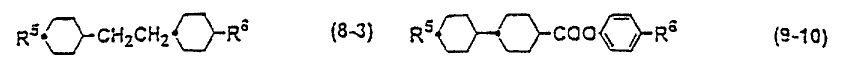

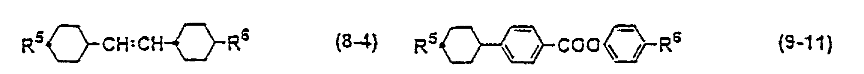

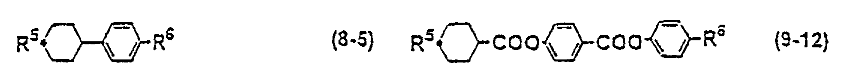

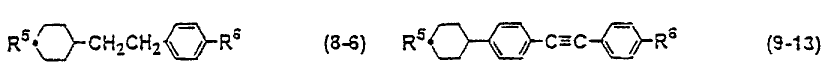

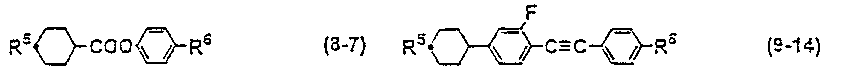

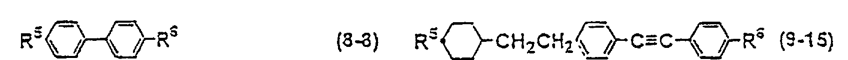

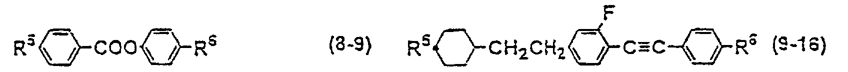

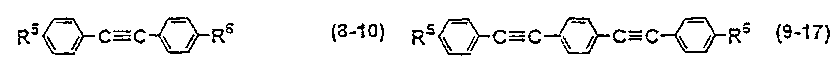

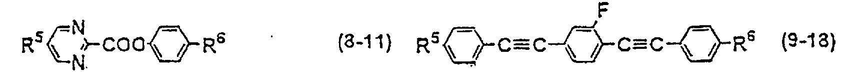

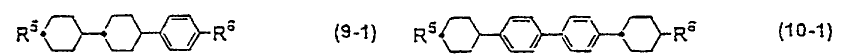

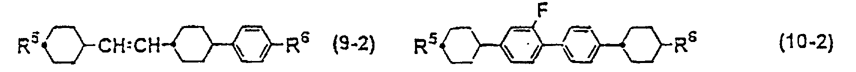

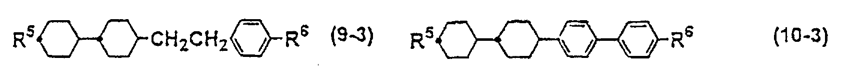

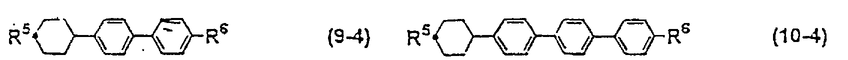

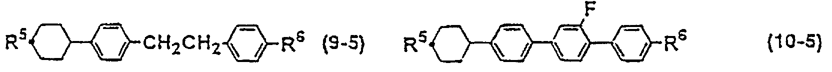

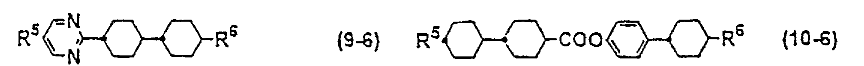

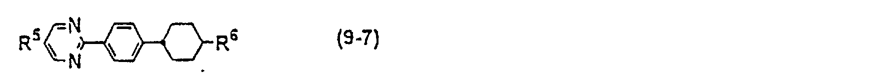

The compounds represented by Formulas (8) to (10) have dielectric anisotropy values whose absolute values are small and are almost neutral ones. The compounds of Formula (8) are mainly used for controlling the viscosity or optical anisotropy value of the resulting liquid crystal composition, while the compounds of Formula (9) and (10) are used for expanding the nematic range, for instance, increasing the clearing point or for controlling the optical anisotropy value.

If the amount of the compounds of Formulas (8) to (10) used increases, the threshold voltage of the resulting liquid crystal composition increases and the viscosity thereof decreases. Therefore, they can be used in a large amount inasmuch as the composition satisfies the requirememt for threshold voltage. When preparing a liquid crystal composition for TFT's, the amount of the compounds of Formulas (8) to (10) is preferably not more than 40% by weight and more preferably not more than 35% by weight based on the total weight of the composition, while when preparing a liquid crystal composition for STN's or TN's, the amount thereof is preferably not more than 70% by weight and more preferably not more than 60% by weight based on the total weight of the composition.

The following are examples of preferred compounds represented by Formulas (11) to (13) which can be used in the liquid crystal composition of the present invention (in these compounds, R⁷ and R⁸ are the same as those defined above):

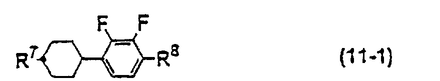

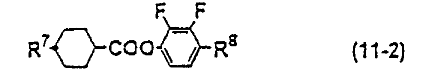

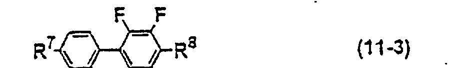

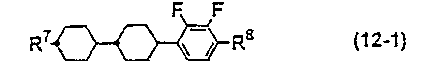

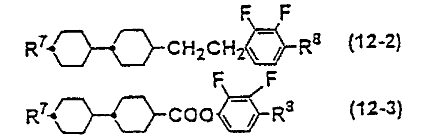

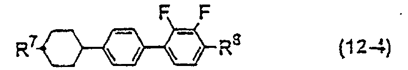

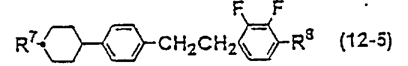

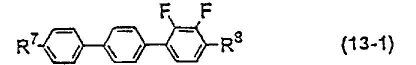

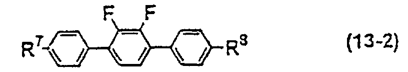

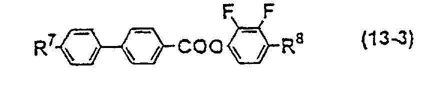

The compounds represented by Formulas (11) to (13) have negative dielectric anisotropy values like the compounds according to the first aspect of the present invention and the preferred embodiments thereof and are used as base compounds for N-type liquid crystal compositions or are used for controlling the dielectric anisotropy of P-type liquid crystal compositions.

Moreover, the compounds of Formula (11) are bicyclic compounds and mainly used for controlling the threshold voltages, viscosities or optical anisotropy values of the resulting compositions. The compounds of Formula (12) are used for expanding the nematic range, for instance, increasing the clearing point or for controlling the optical anisotropy value of the resulting composition. Further the compounds of Formula (13) are used not only for expanding the nematic range, but also for reducing the threshold voltage and for increasing the optical anisotropy value of the resulting composition.

The compounds of Formula (11) to (13) are mainly used in the N-type liquid crystal compositions and if the amount thereof used increases, the threshold voltage of the resulting liquid crystal composition is reduced, while the viscosity thereof is increased. For this reason, these compounds are desirably used in a small amount inasmuch as the liquid crystal composition satisfies the requirement for threshold voltage. However, the absolute value of the negative dielectric anisotropy thereof is not more than 5 and therefore, if the amount thereof used is less than 40% by weight, the resulting display device cannot sometimes be operated at a low voltage. When preparing a liquid crystal composition for N-type TFT's, the amount of the compounds of Formula (11) to (13) is preferably not less than 40% bY weight and more preferably 50 to 95% by weight based on the total weight of the composition. Moreover, the compounds of Formula (11) to (13) are often incorporated into P-type compositions for controlling the elastic constant and the voltage-transmittance curve (V-T curve) of the resulting liquid crystal composition. In this case, the amount of the compounds of Formula (11) to (13) is preferably not more than 30% by weight based on the total weight of the composition.

In addition, the liquid crystal composition of the present invention may in general comprise an optically active compound in order to induce the formation of a helical structure of the liquid crystal composition to thus achieve a desired twist angle and prevent the occurrence of any reverse twist, except for some particular cases such as liquid crystal compositions for OCB (Optically Compensated Birefringence).

Any known optically active compounds used for such purposes may be used in the present invention, but preferred are optically active compounds listed below:

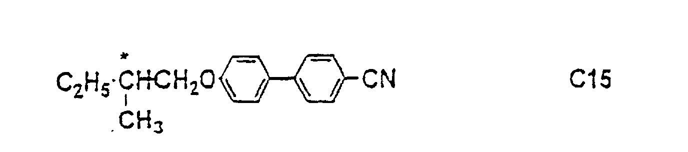

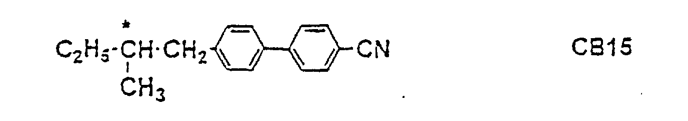

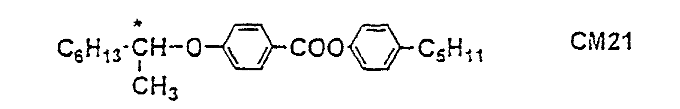

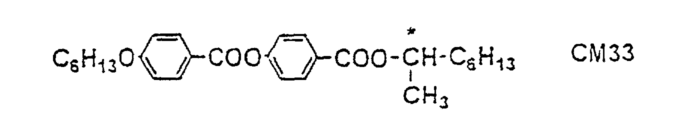

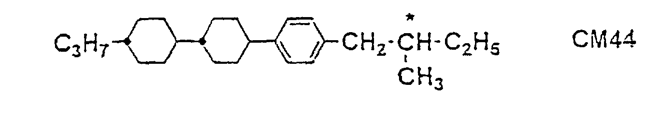

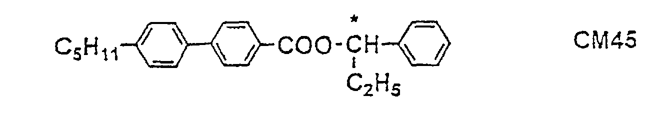

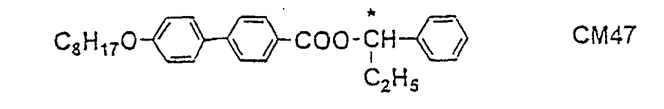

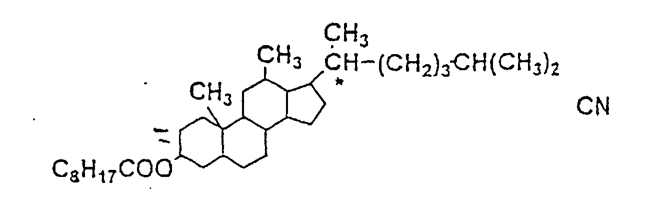

These optically active compounds are usually incorporated into the liquid crystal composition of the present invention to thus control the pitch of twist. The pitch of twist is preferably adjusted to the range of from 40 to 200 µm in case of the liquid crystal compositions for TFT's and TN's. Moreover, it is adjusted to the range of from 6 to 20µm in case of the liquid crystal compositions for STN's and 1.5 to 4 µm for bistable TN's. Moreover, at least two optically active compounds may be added to the composition to control the temperature-dependency of the pitch.

The liquid crystal composition of the present invention may be used as the composition for GH's by addition of a dichroic dye such as merocyanine, styryl, azo, azomethine, azoxy, quinophthalone, anthraquinone, or tetrazine type ones. Alternatively, the composition of the present invention can likewise be used as a liquid crystal composition for polymer-dispersion type liquid crystal display devices (PDLCD) including NCAP's which are produced by encapsulation of a nematic liquid crystal and polymer network liquid crystal display devices (PNLCD) produced by forming a three-dimensional network polymer in the liquid crystal. Moreover, the liquid crystal composition of the present invention may be used in DS's.

The composition of the present invention can be prepared by the commonly used methods. In general, it is prepared by a method wherein various ingredients are dissolved together at a high temperature.

The compounds according to the first aspect of the present invention and the preferred embodiments thereof can easily be prepared by any known organic chemical synthesis technique. An example of the production methods will be given in the following reaction scheme 1:

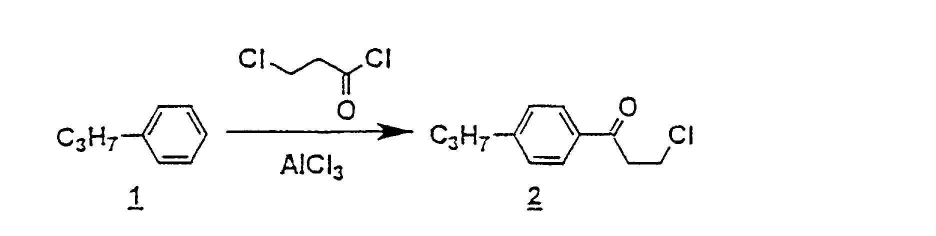

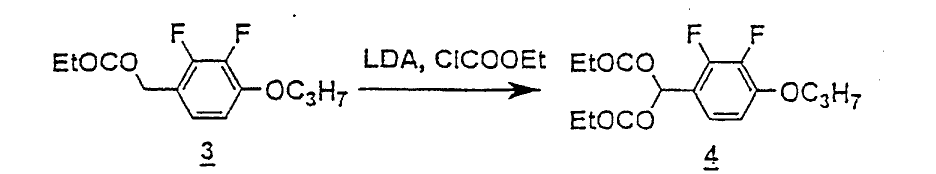

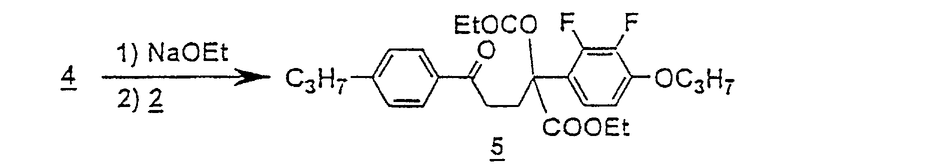

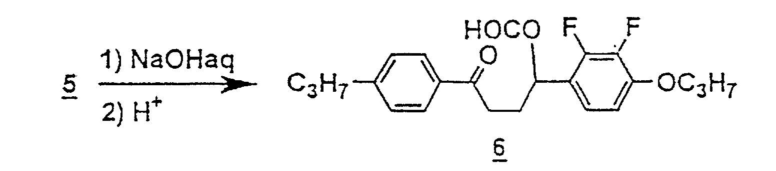

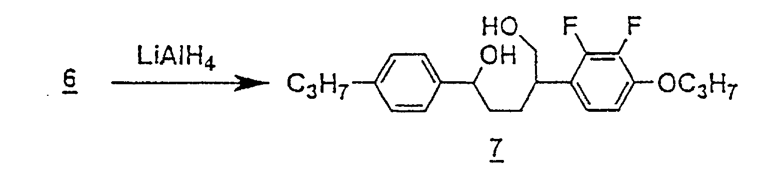

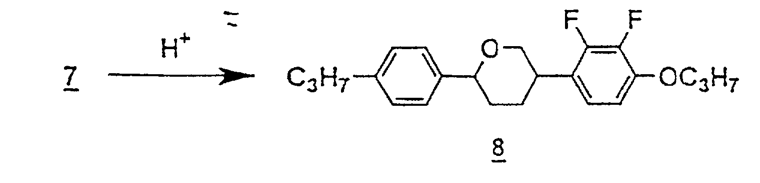

The foregoing route of synthesis will be detailed below.

3-Chloropropionic chloride is added to the compound represented bY Formula 1 through a Friedel-Crafts reaction in the presence of anhydrous aluminum chloride to give the compound of Formula 2.

The compound represented by Formula 3 is reacted with ethyl chlorocarbonate in the presence of LDA (lithium diisopropyl amide) to give the compound of Formula 4.

The compound represented by Formula 4 is reacted with NaOEt (sodium ethoxide) and then reacted with the compound of Formula 2 to give the compound of Formula 5, followed by hydrolyzed with NaOH to give the compound of Formula 6, then reduction with LAH (lithium aluminum hydride) to give the compound of Formula 7 and final dehydration using an acid catalyst to give the intended compound of Formula 8.

The compound of the present invention can likewise be prepared by the method as shown in the following reaction scheme 2. More specifically, an aldehyde derivative 9 is reacted with a bromoacetic acid ester through a Refomatsky reaction (M.W. RATHKE et al., J.O.C., 1970, 35(11), p.3966; J. F. Ruppert et al., J.O.C., 1974, 39(2), p. 269) to give a compound of Formula 10, followed by reduction with a reducing agent such as lithium aluminum hydride (LAH), sodium bis(2-methoxyethoxy)aluminum hydride, diisobutyl aluminum hydride (DIBAL) or sodium borohydride to give a compound of Formula 11. This compound is converted into a compound of Formula 12 by the method of P. PICARD et al. (Synthesis, 550 (1981)) and then the latter is converted into a compound of Formula 15 by the method of M. Yamaguchi et al. (Tetrahedron Lett., 1984, 25(11), p. 1159). Then the compound 15 is reduced to a compound of Formula 16 by DIBAL, followed by hydrosilylation (G.A. Kraus et al., J. Org. Chem., 1981. 46, p. 2417; G.A. Kraus et al., J. Chem. Soc. Chem. Commun., 1568 (1986)) to give an intended compound of Formula 17.

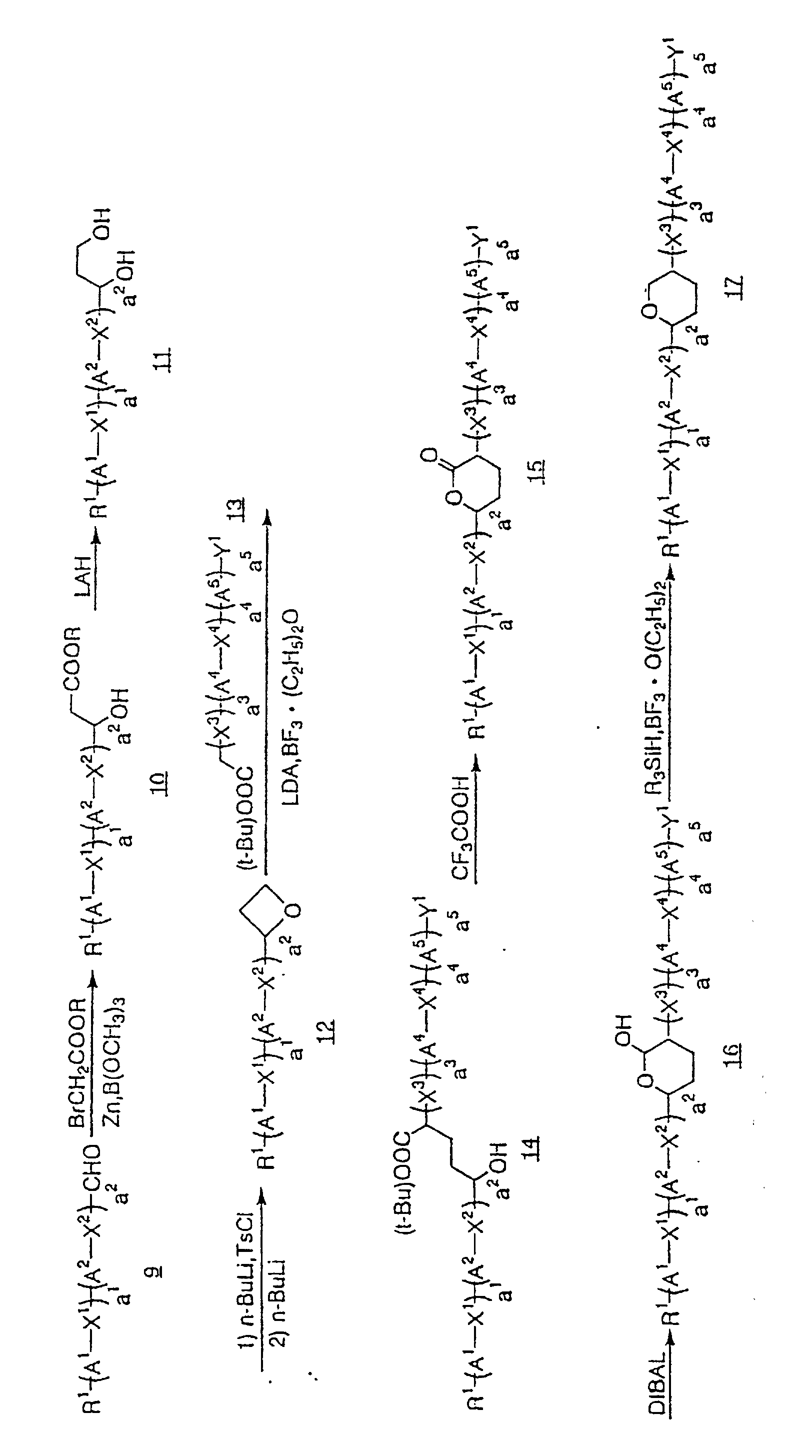

The method of preparing the compound of the present invention and the use thereof will be descrived below in detail with reference to the following working Examples, but the present invention is not restricted to these specific Examples at all. In the description of the following Examples, the symbol N represents the normal concentration (gram eq./l); M represents the molar concentration (mol/l); Δ ε (dielectric anisotropy value) of a liquid crystal compound represents the value obtained by extrapolating the values (as measured at 25 °C) observed for the compositions comprising 85 wt% of the following mother liquid crystal A and 15 wt% of each corresponding compound; and Δn (optical anisotropy value) represents the measured value (as determined at 25 °C) for the composition comprising 85 wt% of the following mother liquid crystal B and 15 wt% of each corresponding compound.

### Composition of Mother Liquid Crystal A

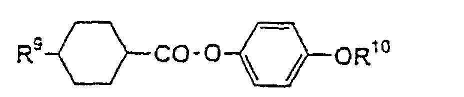

Five kinds of ester compounds represented by the foregoing general formula and whose both terminal alkyl groups (R⁹, R¹⁰) were different from each other were mixed in a ratio specified below to give the desired mother liquid crystal A.

| | | |
|---|---|---|
| R ⁹ = C ₃ H ₇ , | R ¹⁰ = C ₄ H ₉ | 27.6 wt% |
| R ⁹ = C ₄ H ₉ , | R ¹⁰ = C ₂ H ₅ | 20.7 wt% |
| R ⁹ = C ₅ H ₁₁ , | R ¹⁰ = CH ₃ | 20.7 wt% |
| R ⁹ = C ₃ H ₇ , | R ¹⁰ = C ₂ H ₅ | 17.2 wt% |
| R ⁹ = C ₅ H ₁₁ , | R ¹⁰ = C ₂ H ₅ | 13.8 wt% |
| | | 100.0 |

Δ ε = -1.5

### Composition of Mother Liquid Crystal 8

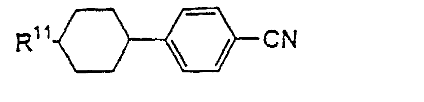

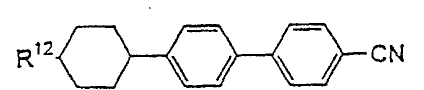

Four kinds of compounds represented by the foregoing general formulas and whose both terminal alkyl groups (R¹¹, R¹²) were different from each other were mixed in a ratio specified below to give the desired mother liquid crystal B.

| | |
|---|---|
| R¹¹ = C₃H₇ | 24.0 wt% |
| R¹¹ = C₅H₁₁ | 36.0 wt% |
| R¹¹ = C₇H₁₅ | 25.0 wt% |
| R¹² = C₅H₁₁ | 15.0 wt% |
| | 100.0 |

Δn = 0.137

### Example 1: Synthesis of 2-(4-n-Propylphenyl)-5-(2,3-Difluoro-4-n-Propyloxyphenyl)Tetrahydropyran (8)

### First Step: Synthesis of 1-n-Propyl-4-(1-Oxo-3-Chloro-n-Propyl)-Benzene (2)

A mixture of propylbenzene (1) (120.2g, 1.00 mole), 3-chloropropionic chloride (139.7g, 1.10 mole), anhydrous aluminum chloride (146.7g, 1.10 mole) and methylene chloride (1200 ml) was reacted at room temperature for 12 hours, in a nitrogen gas atmosphere. After completion of the reaction, the reaction solution was poured into a 1N dilute hydrochloric acid solution (1500 ml) with cooling, followed by extraction with toluene (2000 ml). The resulting organic phase was washed with water (1500 ml) and dried over anhydrous magnesium sulfate. The solvent was distilled off under reduced pressure, the resulting residue was purified by silica gel column chromatography (eluent: 1:1 hexane/toluene mixture) to give the title compound, 1-n-propyl-4-(1-oxo-3-chloro-n-propyl)benzene (2) (84.28g, 0.400 mole; yield 40%). MS/211 (M ⁺ ).

### Second Step: Synthesis of Diethyl 2-(2,3-Difluoro-4-n-Propyloxyphenyl)Malonate (4)

To a mixture of ethyl 2,3-difluoro-4-propyloxyphenyl acetate (3) (258.3g, 1.00 mole) and diethyl ether (800 ml), there was dropwise added a solution of lithium diisopropylamide (107.1g, 1.00 mole) in tetrahydrofuran (500 ml) at -78 °C over 30 minutes and they were reacted at that temperature for one hour. A solution of ethyl chlorocarbonate (108.5g, 1.00 mole) in diethyl ether (300 ml) was dropwise added, over 30 minutes, to the reaction solution while maintaining the solution at that temperature, followed by gradually raising the temperature and reaction at 0°C for 12 hours. After completion of the reaction, water (1000 ml) was added to the reaction solution, followed by extraction with hexane (1000 ml). The resulting organic phase was washed with water (800 ml) and dried over anhydrous magnesium sulfate. The solvent was distilled off under reduced pressure and the resulting residue was purified by silica gel column chromatography (eluent: hexane) to give the title compound, diethyl 2-(2,3-difluoro-4-propyloxyphenyl)malonate (4) (218.2g, 0.661 mole; Yield 66%). MS/330 (M ⁺ )

### Third Step: Synthesis of Ethyl 2-Ethoxycarbonyl-2-(2,3-Difluoro-4-n-Propyloxyphenyl)-5-Oxo-5-(4-n-Propylphenyl)Valerate (5)

To a solution of diethyl 2-(2,3-difluoro-4-propyloxyphenyl) malonate (4) (132.0g, 0.400 mole) obtained in the second step in diethyl ether (500 ml), there was dropwise added a solution of NaOEt (40.83g, 0.600 mole) in diethyl ether (300 ml), at -10 °C, over 30 minutes, followed by reaction for 2 hours. Thereafter, a solution of 1-n-propyl-4-(1-oxo-3-chloro-n-propyl)benzene (2) (84.20g, 0.400 mole) obtained in the first step in diethyl ether (200 ml) was dropwise added to the reaction solution at that temperature over 30 minutes, followed by reaction at room temperature for 8 hours. After completion of the reaction, water (400 ml) was added to the reaction mixture and then the mixture was extracted with toluene (400 ml). The resulting organic phase was dried over anhydrous magnesium sulfate. The solvent was distilled off under reduced pressure, the resulting residue was purified by silica gel column chromatography (eluent: 5:1 hexane/ethyl acetate mixture) to give the title compound, ethyl 2-ethoxycarbonyl-2-(2,3-difluoro-4-n-propyloxyphenyl)-5-oxo-5-(4-n-propylphenyl)valerate (5) (86.38g, 0.171 mole; yield 43%). MS/505 (M ⁺ ).

### Fourth Step: Synthesis of 2-(2,3-Difluoro-4-n-Propyloxyphenyl)-5-Oxo-5-(4-n-Propylphenyl)Valeric Acid (6)

Ethyl 2-ethoxycarbonyl-2-(2,3-difluoro-4-n-propyloxyphenyl)-5-oxo-5-(4-n-propylphenyl)valerate (5) (85.72g, 0.170 mole) obtained in the third step was dropwise added to a solution of sodium hydroxide (8.00g, 0.200 mole) in water/ethanol (1:1) mixed solvent (200 ml) with ice-cooling. After heating the reaction solution under reflux for one hour, the solution was cooled to room temperature, followed by adjusting the pH thereof to about 2 by addition of 1N-HCl and again heating the solution under reflux. After completion of the reaction, the reaction solution was concentrated under reduced pressure and then extracted with diethyl ether (600 ml). The resulting organic phase was dried over anhydrous magnesium sulfate. The solvent was distilled off under reduced pressure, the resulting residue was purified by recrystallization (solvent: acetic acid) to give the title compound, 2-(2,3-difluoro-4-n-propyloxyphenyl)-5-oxo-5-(4-n-propylphenyl)valeric Acid (6) (38.02g, 0.0940 mole; yield 55%). MS/404 (M ⁺ ).

### Fifth Step: Synthesis of 1-(4-n-Propylphenyl)-4-(2,3-Difluoro-4-n-Propyloxyphenyl)Pentane-1,5-Diol (7)

A solution of 2-(2,3-difluoro-4-n-propyloxyphenyl)-5-oxo-5-(4-n-propylphenyl)valeric Acid (6) (36.98g, 0.0939 mole) obtained in the fourth step in ethanol (200 ml) was cooled and LAH (3.57g, 0.0940 mole) was added thereto at -10°C. The temperature of the reaction solution was gradually raised up to room temperature, followed by reaction at that temperature for 8 hours. After completion of the reaction, water (100 ml) was added to the reaction solution with ice-cooling and then a 1N dilute hydrochloric acid solution (50 ml) was added. After completion of the reaction, the reaction solution was concentrated under reduced pressure and extracted with ethyl acetate (400 ml). The resulting organic phase was dried over anhydrous magnesium sulfate. The solvent was distilled off under reduced pressure and the resulting residue was purified by silica gel column chromatography (eluent: toluene) to give the title compound, 1-(4-n-propylphenyl)-4-(2,3-difluoro-4-n-propyloxyphenyl)pentane-1,5-diol (7) (28.64g, 0.0730 mole; yield 78%). MS/392 (M ⁺ ).

### Sixth Step: Synthesis of 2-(4-n-Propylphenyl)-5-(2,3-Difluoro-4-n-Propyloxyphenyl)Tetrahydropyran (8)

A mixture of 1-(4-n-propylphenyl)-4-(2,3-difluoro-4-n-propyloxyphenyl)pentane-1,5-diol (7) (28.55g, 0.0727 mole) obtained in the fifth step, p-toluenesulfonic acid monohydrate (0.691g, 0.0036 mole) and toluene (100 ml) was heated under reflux for 5 hours while distilling off water. After completion of the reaction, the reaction solution was cooled to room temperature and then washed with water (100 ml) two times. The resulting organic phase was dried over anhydrous magnesium sulfate. The solvent was distilled off under reduced pressure and the resulting residue was purified by silica gel column chromatography (eluent: hexane) and then recrystallization (solvent: hexane) to give the title compound, 2-(4-n-propylphenyl)-5-(2,3-difluoro-4-n-propyloxyphenyl)tetrahydropyran (8) (9.34g, 0.0249 mole; yield 34%). MS/374 (M ⁺ ), Δε = -4.4; Δn=0.138.

### Example 2: Synthesis of 2-(Trans-4-Propylcyclohexyl)-5-(2,3-Difluoro -4-Ethoxyphenyl)Tetrahydropyran (24)

### First Step: Synthesis of Ethyl 3-Hydroxy-3-(Trans-4-Propylcyclohexyl)Propanoate (18)

To a mixture of trans-4-propylcyclohexyl carbaldehyde (50.0g, 0.32 mole), zinc powder (29.6g), trimethoxy borane (96.0g, 0.92 mole) and tetrahydrofuran (THF; 500 ml), there was dropwise added, in a nitrogen gas atmosphere, a solution of ethyl bromoacetate (75.8g, 0.45 mole) in THF (150 ml) over one hour and they were reacted at 50°C for 2 hours. The reaction solution was ice-cooled, followed by addition of a dilute NH₄Cl solution (1000 ml), and extraction of the reaction product with diethyl ether (300 ml). The resulting organic phase was washed with water (2x300 ml), dried over anhydrous magnesium sulfate and the solvent was distilled off under reduced pressure. The resulting residue was distilled (127.5 to 130.0 °C/1.1mmHg) to give the title compound, ethyl 3-hydroxy-3-(trans-4-propylcyclohexyl) propanoate (18) (59.8g, yield 76%).

### Second Step: Synthesis of 3-Hydroxy-3-(Trans-4-Propylcyclohexyl)-Propanol (19)

To a mixture of LAH (9.3g, 0.24 mole) and THF (300 ml), there was dropwise added a solution of ethyl 3-hydroxy-3-(trans-4-propylcyclohexyl)propanoate (18) (59.8g, 0.24 mole) obtained in the first step in THF (200 ml) at room temperature over 30 minutes in a nitrogen gas atmosphere, followed by reaction at that temperature for 2 hours. After dropwise addition of a 2N HCl solution (150 ml) to the reaction solution, the reaction product was extracted with ethyl acetate (300 ml). The resulting organic phase was washed with water (2x300 ml), dried over anhydrous magnesium sulfate and the solvent was distilled off under reduced pressure. The resulting residue was distilled (145.0 to 146.5 °C/1.2mmHg) to give the title compound, 3-hydroxy-3-(trans-4-propylcyclohexyl)propanol (19) (41.6g, yield 84%).

### Third Step: Production of 2-(Trans-4-Propylcyclohexyl)Oxetane (20)

To a solution of 3-hydroxy-3-(trans-4-propylcyclohexyl)propanol (19) (20.0g, 0.10 mole) prepared in the second step in THF (150 ml), there was dropwise added n-BuLi (1.66M THF solution, 60 ml; corresponding to 0.10 mole) in a nitrogen gas atmosphere while maintaining the temperature to not more than -20°C, followed by reaction thereof at that temperature for one hour, dropwise addition of a solution of p-toluenesulfonyl chloride (19.0g, 0.10 mole) in THF (60 ml) while maintaining the temperature of the solution to not more than 0 °C, and reaction at that temperature for one hour. Then n-BuLi (1.66M THF solution, 60 ml; corresponding to 0.10 mole) was dropwise added to the reaction solution while maintaining the temperature of the solution to not more than 0 °C, followed by reaction at that temperature for one hour and reflux for one hour. The reaction was stopped by addition of water (100 ml) to the reaction solution and the reaction product was extracted with heptane (100 ml). The resulting organic phase was washed with water (2x200 ml), dried over anhydrous magnesium sulfate and the solvent was distilled off under reduced pressure. The resulting residue was distilled (74.0 to 76.5 °C /0.7mmHg) to give the title compound, 2-(trans-4-propylcyclohexyl) oxetane (20) (13.9g, yield 76%).

### Fourth Step: Production of Tert-Butyl 2-(2,3-Difluoro-4-Ethoxyphenyl)-5-Hydroxy-5-(Trans-4-Propylcyclohexyl)-Pentanoate (21)

To a solution of tert-butyl 1-(2,3-difluoro-4-ethoxyphenyl) acetate (44.8g, 0.16 mole) in THF (100 ml), there was dropwise added lithium diisopropylamide (2M toluene solution, 83 ml: corresponding to 0.16 mole) in a nitrogen gas atmosphere while maintaining the temperature of the solution to not more than -60 °C, followed by reaction at that temperature for 30 minutes. To this reaction solution, there were dropwise added a solution of 2-(trans-4-propylcyclohexyl)oxetane (20) (10.0g, 0.05 mole) obtained in the third step in THF (40 ml) and boron trifluoride-diethtyl ether complex (19 ml: corresponding to 0.15 mole) in a nitrogen gas atmosphere while maintaining the temperature of the solution to not more than -60°C, followed by reaction at that temperature for 30 minutes and then at room temperature for additional 8 hours.

To this reaction mixture, there was added a dilute NH₄Cl solution (100 ml) and then the product was extracted with diethyl ether (200 ml). The resulting organic phase was washed with water (2x150 ml), dried over anhydrous magnesium sulfate and the solvent was distilled off under reduced pressure. The resulting residue was purified by silica gel column chromatography (eluent: 9:1 toluene/ethyl acetate mixture) to give the title compound, tert-butyl 2-(2,3-difluoro-4-ethoxyphenyl)-5-hydroxy-5-(trans-4-propylcyclohexyl) pentanoate (21) (23.2g, yield 93%).

### Fifth Step: Production of 3-(2,3-Difluoro-4-Ethoxyphenyl)-6-(Trans-4-Propylcyclohexyl)Tetrahydro-2-Pyrone (22)

To a solution of tert-butyl 2-(2,3-difluoro-4-ethoxyphenyl)-5-hydroxy-5-(trans-4-propylcyclohexyl)pentanoate (21) (23.2g, 0.05 mole) obtained in the fourth step in dichloromethane (100 ml), there was dropwise added, with ice-cooling, trifluoroacetic acid (30 ml: corresponding to 0.25 mole), followed by reaction at room temperature for 5 hours. This reaction mixture was poured into water (100 ml) and the reaction product was extracted with dichloromethane (100 ml). The resulting organic phase was washed with water (2x150 ml), dried over anhydrous magnesium sulfate and the solvent was distilled off under reduced pressure. The resulting residue was purified by silica gel column chromatography (eluent: 9:1 toluene/ethyl acetate mixture) and then recrystallization from a 1:1 heptane/ethyl acetate mixed solvent to give the title compound, 3-(2,3-difluoro-4-ethoxyphenyl)-6-(trans-4-propylcyclohexyl)tetrahydro-2-pyrone (22) (5.8g. yield 29%).

### Sixth Step: Production of 2-Hydroxy-3-(2,3-Difluoro-4-Ethoxyphenyl)-6-(Trans-4-Propylcyclohexyl)Tetrahydropyran (23)

To a solution of 3-(2,3-difluoro-4-ethoxyphenyl)-6-(trans-4-propylcyclohexyl)tetrahydro-2-pyrone (22) (4.6g, 0.01 mole) obtained in the fifth step in toluene (100 ml), there was dropwise added DIBAL (1.01M toluene solution, 24 ml; corresponding to 0.02 mole) in a nitrogen gas atmosphere while maintaining the temperature of the solution to not more than -60°C, followed by reaction at that temperature for 3 hours. After completion of the reaction, the reaction solution was poured into acetic acid (50 ml) and the reaction product was extracted with diethyl ether (100 ml). The resulting organic phase was washed with water (2x150 ml), dried over anhydrous magnesium sulfate and the solvent was distilled off under reduced pressure to give the title compound, 2-hydroxy-3-(2,3-difluoro-4-ethoxyphenyl)-6-(trans-4-propylcyclohexyl)tetrahydropyran (23) (5.0g; yield: quantitative). This compound was used in the subsequent reaction without further purification.

### Seventh Step: Production of 2-(Trans-4-Propylcyclohexyl)-5-(2,3-Difluoro-4-Ethoxyphenyl)Tetrahydropyran (24)

To a solution obtained by dissolving 2-hydroxy-3-(2,3-difluoro-4-ethoxyphenyl)-6-(trans-4-propylcyclohexyl)tetrahydropyran (23) (5.0g, 0.01 mole) obtained in the sixth step and boron trifluoride-diethyl ether complex (3.9 ml; corresponding to 0.03 mole) in dichloromethane (40 ml), there was dropwise added triethylsilane (5.1 ml; corresponding to 0.03 mole) in a nitrogen gas atmosphere while maintaining the temperature of the solution to not more than -60°C, followed by reaction at that temperature for 3 hours and then at room temperature for additional 2 hours. The reaction solution was then poured into water (50 ml) and the reaction product was extracted with diethyl ether (50 ml). The resulting organic phase was washed with water (2x100 ml), dried over anhydrous magnesium sulfate and the solvent was distilled off under reduced pressure. The resulting residue was purified by silica gel column chromatography (eluent: 20: 1 heptane/ethyl acetate mixture) and subsequent recrystallization from ethanol to give the title compound, 2-(trans-4-propylcyclohexyl)-5-(2,3-difluoro-4-ethoxyphenyl)tetrahydropyran (24) (0.9g, yield 20%). This compound could form a liquid crystal phase and the transition point thereof was found to be C 65.2-65.5 N 140.0 Iso. MS/366 (M ⁺ ); Δε = -7.3; Δn=0.107.

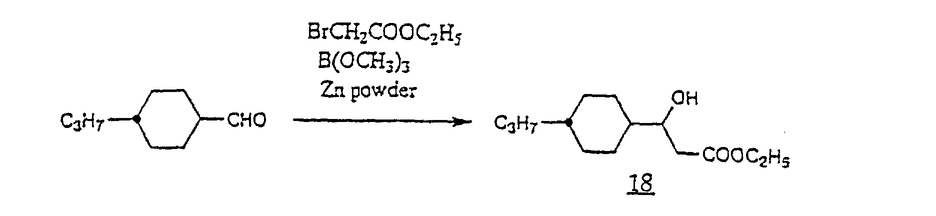

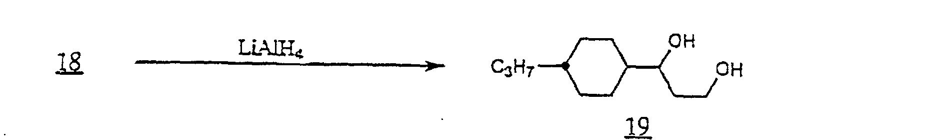

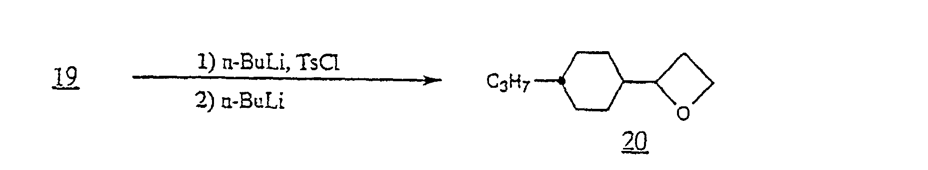

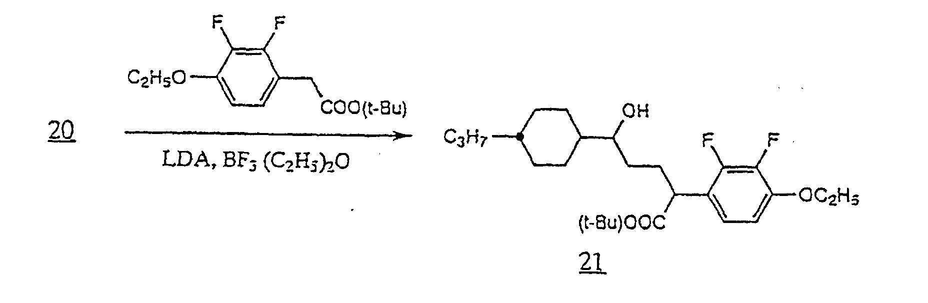

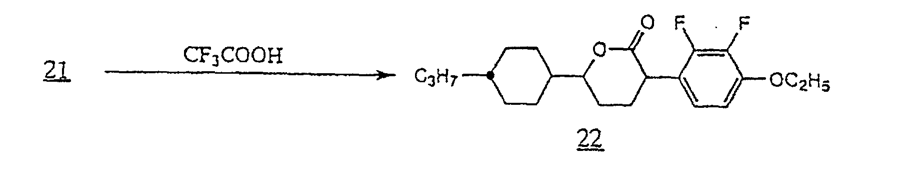

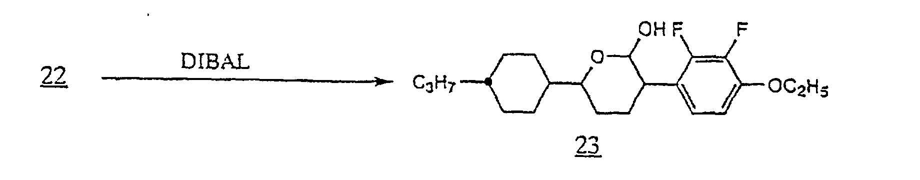

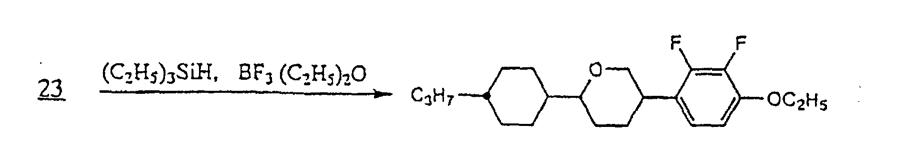

The following compounds can be prepared according to the method described in Examples 1 and 2.

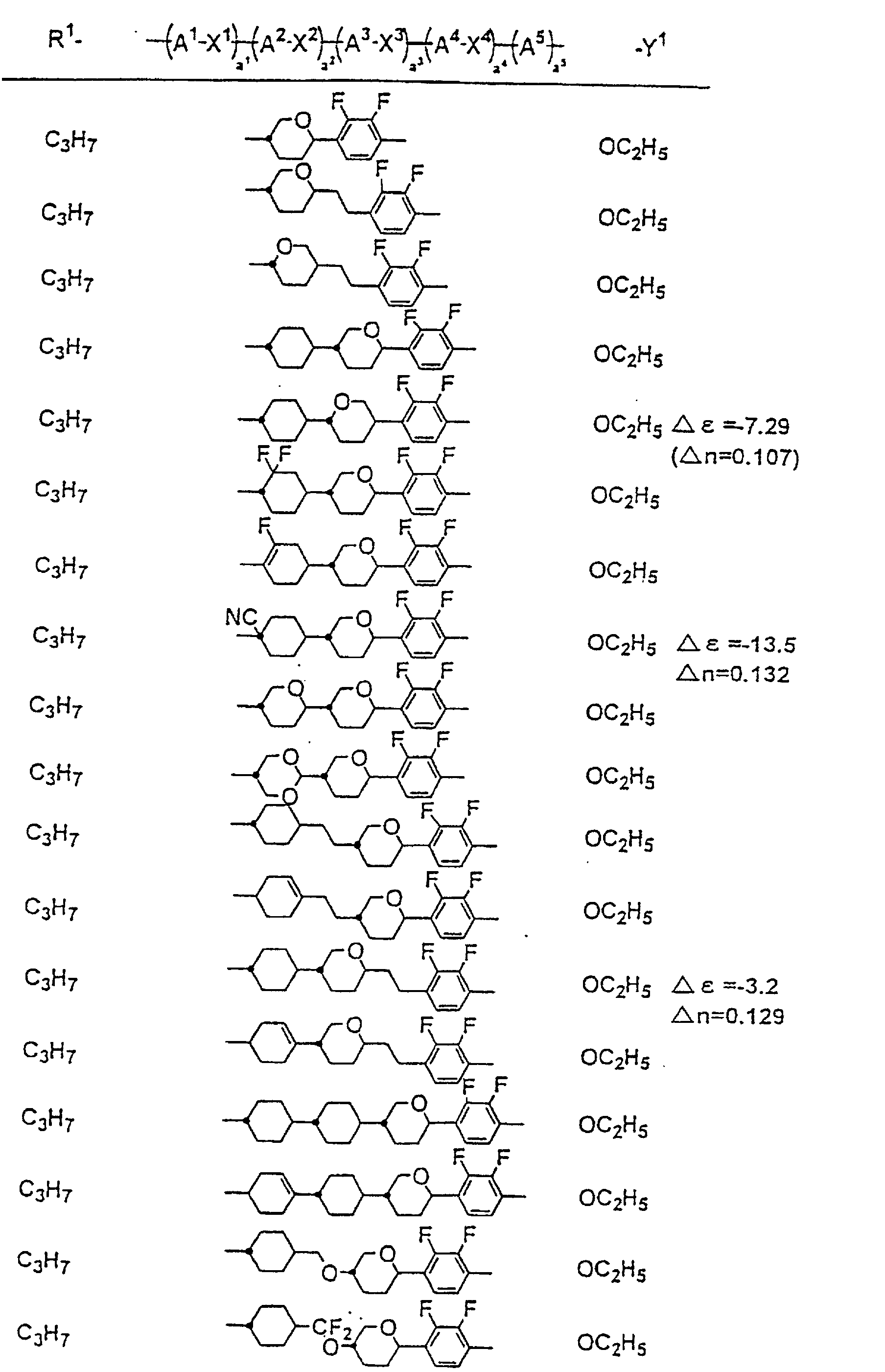

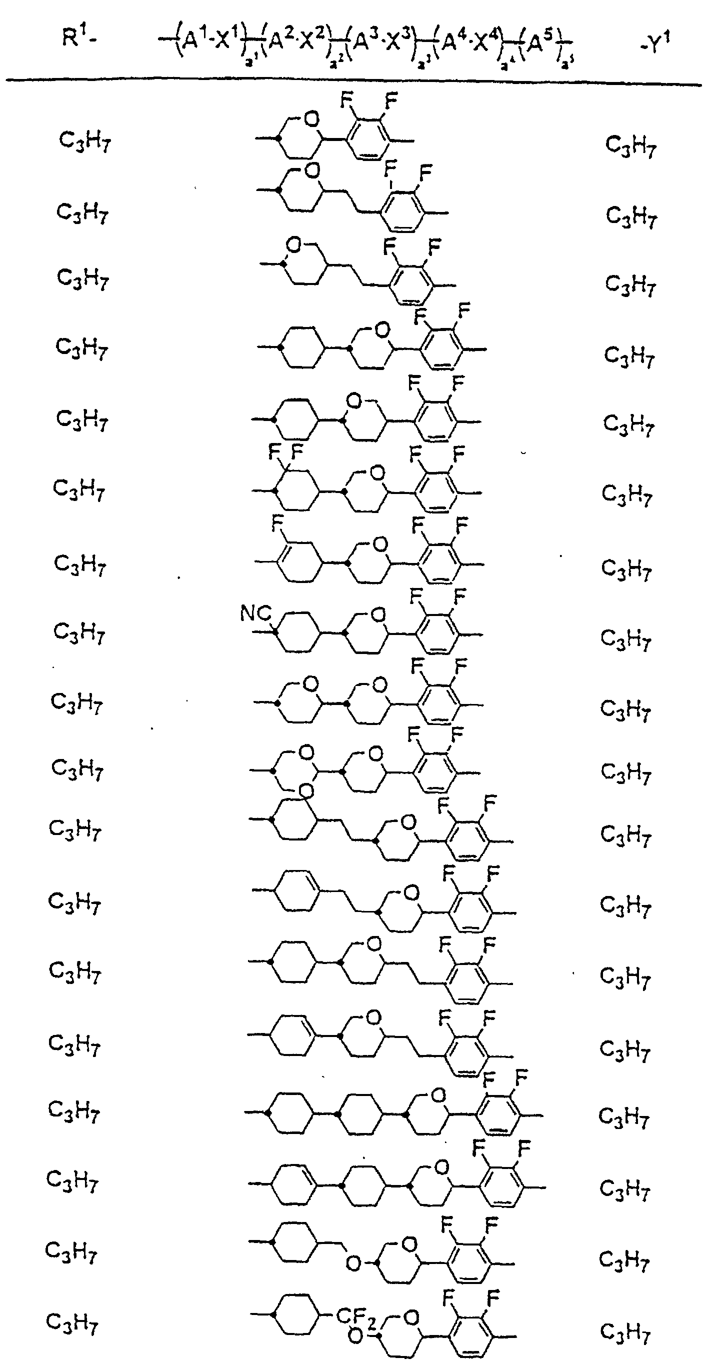

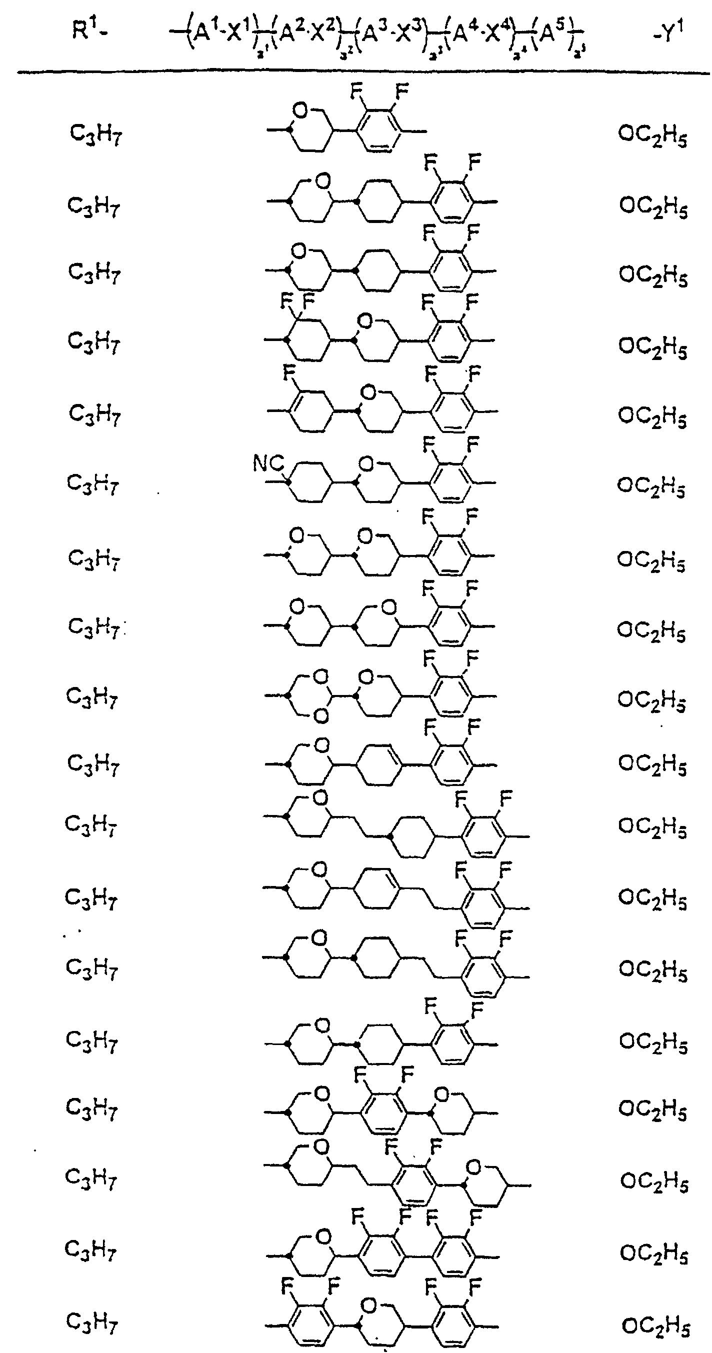

As has been described above in detail, the present invention can provide a liquid crystal compound which has a negative dielectric anisotropy value whose absolute value is large, a low viscosity, a controlled optical anisotropy value, a high specific resistance and a high voltage-holding ratio and which is stable against heat and irradiation with ultraviolet rays, as well as a liquid crystal composition containing the compound and a liquid crystal display device produced using the composition.

## Claims

1. A liquid crystal compound represented by the following general formula (2):
R¹-(A¹-X¹)ₐ₁-(A²-X²)ₐ₂-(A³-X³)ₐ₃-(A⁴-X⁴)ₐ₄-(A⁵)ₐ₅-Y¹ (2)
wherein R¹ and Y¹ each independently represents an alkyl group having 1 to 20 carbon atoms, a hydrogen atom, a halogen atom, a cyano group, a cyanate residue, an isocyano group or an isothiocyanate residue, provided that at least one of the methylene groups present in the alkyl group, which are not adjacent to one another, may be substituted with an oxygen, sulfur or nitrogen atom, -C≡C-, a silylene group wherein at least one hydrogen atom may be substituted with an alkyl group having 1 to 10 carbon atoms, or a vinylene group, or that at least one hydrogen atom of the alkyl group may be substituted with a fluorine or chlorine atom or that these alkyl groups may be optically active ones; X¹, X², X³ and X⁴ each independently represents a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC≡C-, or -C ≡CCH=CH-; the rings A¹, A², A³, A⁴ and A⁵ each independently represents a trans-cyclohexane-1,4-diyl, cyclohexa-1-ene-1,4-diyl, 1,4-phenylene, bic yclo[1.1.1]pentane-1,3-diyl, tetrahydropyran-2,5-diyl group, or a ring represented by the following formula (1): 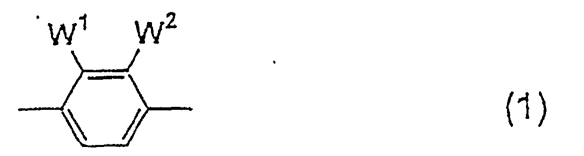 wherein W¹ and W² each independently represents a fluorine atom, provided that at least one of these rings A¹, A², A³, A⁴ and A⁵ represent a tetrahydropyran-2,5-diyl ring and at least one of them represents a ring represented by Formula (1), that the carbon atoms constituting these rings may be substituted with a nitrogen, oxygen or sulfur atom and that each hydrogen atom present on the rings may be substituted with a halogen atom or a cyano group; a¹, a², a³, a⁴ and a⁵ each independently represents 0 or 1, provided that they satisfy the relation: 4≧ a¹+a²+a³+a⁴+a⁵ ≧ 2; and each atom constituting the compound may be substituted with its isotope.

2. The liquid crystal compound of claim 1 wherein the ring represented by Formula (1) is 2,3-difluorobenzene-1,4-diyl.

3. A liquid crystal composition comprising at least two components including at least one liquid crystal compound as set forth in claim 1 or 2.

4. A liquid crystal composition comprising at least one liquid crystal compound as set forth in claim 1 or 2 as a first component and at least one compound selected from the group consisting of those represented by the following general formulas (3), (4) and (5) as a second component: 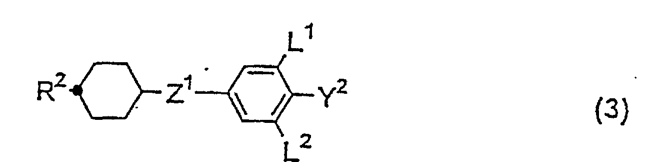 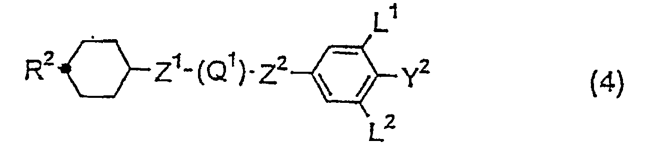 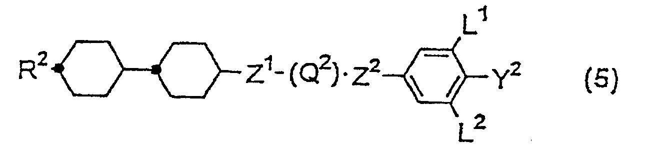 wherein R² represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y² represents a fluorine or chlorine atom, -OCF₃, -OCF₂H, -CF₃, -CF₂H, -CFH₂, -OCF₂CF₂H or -OCF₂CFHCF₃; L¹ and L² each independently represents a hydrogen or fluorine atom; Z¹ and Z² each independently represents -(CH₂)₂-, -(CH₂)₄-, -COO-, -CF₂O-, -OCF₂-, -CH=CH- or a single bond; the ring Q¹ represents a trans-cyclohexane-1,4-diyl or 1,3-dioxane-2,5-diyl group, or a 1,4-phenylene group whose hydrogen atom may be substituted with a fluorine atom; the ring Q² represents a trans-cyclohexane-1,4-diyl group, or a 1,4-phenylene group whose hydrogen atom may be substituted with a fluorine atom; and each atom constituting the compound may be substituted with its isotope.

5. A liquid crystal composition comprising at least one liquid crystal compound as set forth in claim 1 or 2 as a first component and at least one compound selected from the group consisting of those represented by the following general formulas (6) and (7) as a second component: 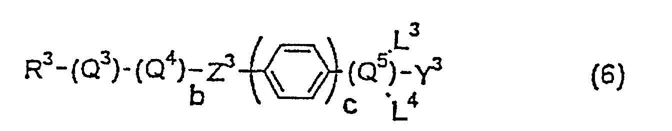 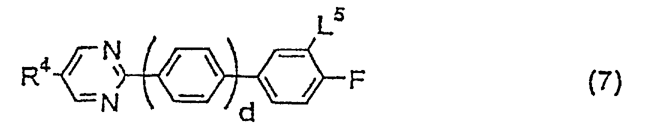 wherein R³ and R⁴ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y³ represents a cYano group or -C ≡C-CN; the ring Q³ represents a trans-cyclohexane-1,4-diyl, 1,4-phenylene, 1,3-dioxane-2,5-diyl or pyrimidine-2,5-diyl group; the ring Q⁴ represents a trans-cyclohexane-1,4-diyl or pyrimidine-2, 5-diyl group, or a 1,4-phenylene group whose hydrogen atom may be substituted with a fluorine atom; the ring Q⁵ represents a transcyclohexane-1,4-diyl or 1,4-phenylene group; Z³ represents -(CH₂)₂-, -COO- or a single bond; L³, L⁴ and L⁵ each independently represents a hydrogen or fluorine atom; b, c and d each independently represents 0 or 1; and each atom constituting the compound may be substituted with its isotope.

6. A liquid crystal composition comprising at least one liquid crystal compound as set forth in claim 1 or 2 as a first component; at least one compound selected from the group consisting of those represented by the general formulas (3), (4) and (5) as a second component; and at least one compound selected from the group consisting of those represented by the following general formulas (8), (9) and (10) as a third component: 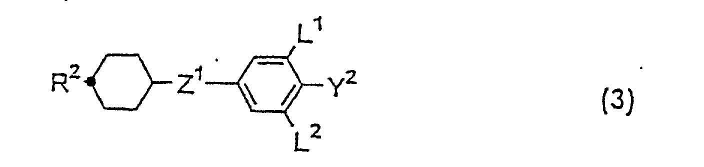 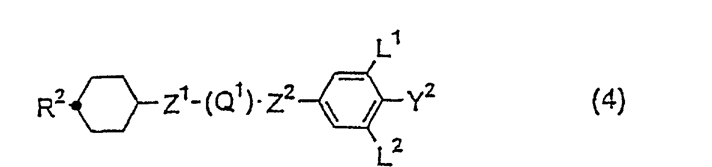 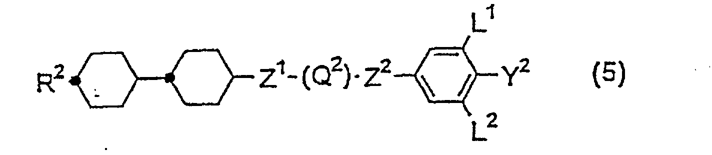
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9) 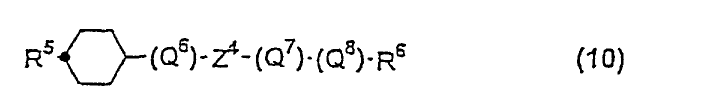
wherein R⁵ and R⁶ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁶, Q⁷ and Q⁸ each independently represents a trans-cyclohexane-1,4-diyl or pyrimidine-2,5-diyl group, or a 1,4-phenylene group whose hydrogen atom may be substituted with a fluorine atom; Z⁴ and Z⁵ each independently represents -C≡C-, -COO-, -(CH₂)₂-, -CH=CH- or a single bond; and each atom constituting the compound may be substituted with its isotope.

7. A liquid crystal composition comprising at least one liquid crystal compound as set forth in claim 1 or 2 as a first component and at least one compound selected from the group consisting of those represented by the following general formulas (11), (12) and (13) as a second component: 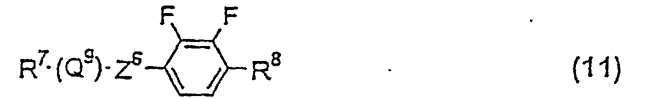 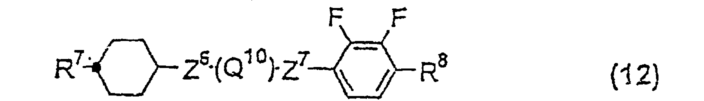 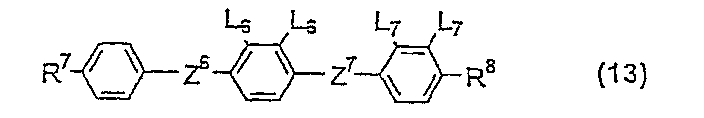 wherein R⁷ and R⁸ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁹ and Q¹⁰ each independently represents a trans-cyclohexane-1,4-diyl or 1,4-phenylene group; L⁶ and L⁷ each independently represents a hydrogen or fluorine atom, provided that L⁸ and L⁷ do not simultaneously represent a hydrogen atom; Z⁶ and Z⁷ each independently represents -(CH₂)₂-, -COO- or a single bond; and each atom constituting the compound may be substituted with its isotope.

8. A liquid crystal composition comprising at least one liquid crystal compound as set forth in claim 1 or 2 as a first component; at least one compound selected from the group consisting of those represented by the general formulas (8), (9) and (10) as a second component; and at least one compound selected from the group consisting of those represented by the general formulas (11), (12) and (13) as a third component:
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

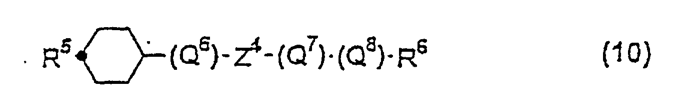

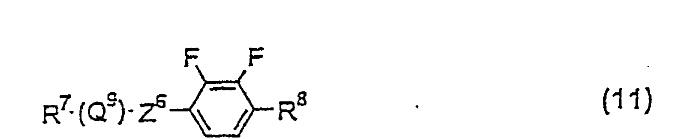

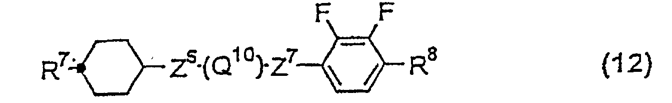

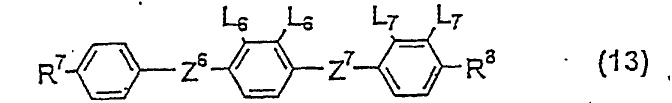

9. A liquid crystal composition comprising at least one liquid crystal compound as set forth in claim 1 or 2 as a first component; at least one compound selected from the group consisting of those represented by the general formulas (6) and (7) as a second component; and at least one compound selected from the group consisting of those represented by the general formulas (8), (9) and (10) as a third component: 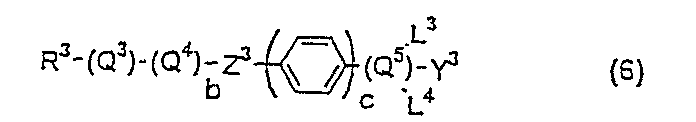 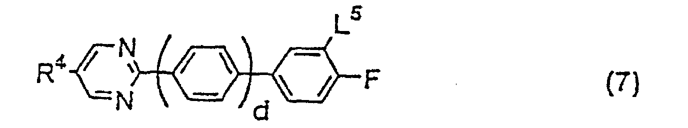
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

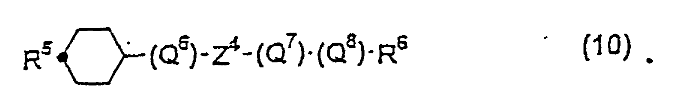

10. A liquid crystal composition comprising at least one liquid crystal compound as set forth in claim 1 or 2 as a first component; at least one compound selected from the group consisting of those represented by the general formulas (3), (4) and (5) as a second component; at least one compound selected from the group consisting of those represented by the general formulas (6) and (7) as a third component; and at least one compound selected from the group consisting of those represented by the general formulas (8), (9) and (10) as a fourth component: 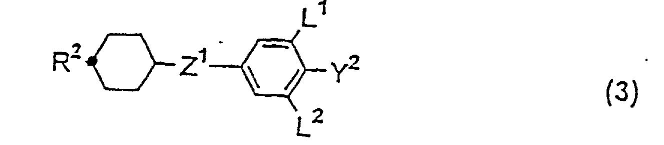 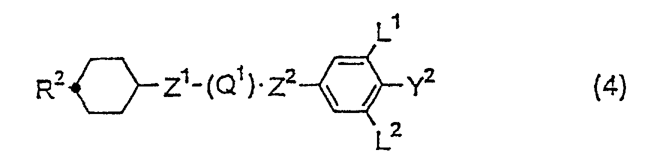 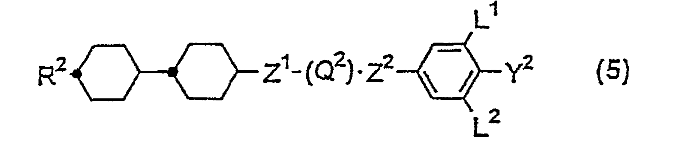 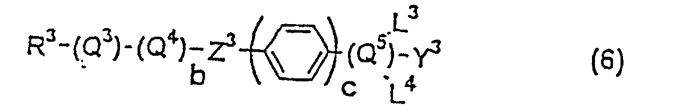 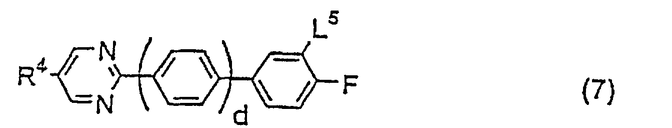
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

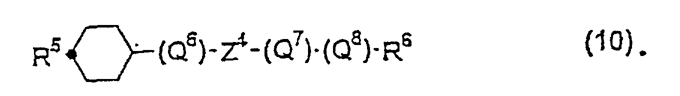

11. A liquid crystal composition comprising at least one optically active compound in addition to one of the liquid crystal compositions as set forth in any one of claims 3 to 10.

12. A liquid crystal display device constituted by the use of one of 3 the liquid crystal compositions as set forth in any one of claims 3 to 11.

## Patentansprüche

1. Flüssigkristallverbindung, dargestellt durch die folgende allgemeine Formel (2):
R¹-(A¹-X¹)ₐ₁-(A²-X²)ₐ₂-(A³-X³)ₐ₃-(A⁴-X⁴)ₐ₄-(A⁵)ₐ₅-Y¹ (2)
wobei R¹ und Y¹ jeweils unabhängig eine Alkylgruppe mit 1 bis 20 Kohlenstoffatomen, ein Wasserstoffatom, ein Halogenatom, eine Cyangruppe, ein Cyanatrest, eine Isocyangruppe oder einen Isothiocyanatrest darstellt, mit der Maßgabe, daß mindestens eine der in der Alkylgruppe vorliegenden Methylengruppen, die nicht miteinander benachbart sind, mit einem Sauerstoff-, Schwefel- oder Stickstoffatom, -C≡C-, einer Silylengruppe, wobei mindestens ein Wasserstoffatom mit einer Alkylgruppe mit 1 bis 10 Kohlenstoffatomen substituiert sein kann, oder einer Vinylengruppe substituiert sein kann, oder daß mindestens ein Wasserstoffatom der Alkylgruppe mit einem Fluor- oder Chloratom substituiert sein kann, oder daß diese Alkylgruppen optisch aktiv sein können, X¹, X², X³ und X⁴ jeweils unabhängig eine Einfachbindung, -(CH₂)₂-, -CH=CH-, -C=C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, CF₂CH₂-, -(CF₂)₂-, -(CF₂)₄-, -(CH₂)₂COO-, -OCO(CH₂)₂-, -CH=CHCOO-, -OCOCH=CH-, -CH=CHC≡C- oder -C≡CCH=CH- darstellt, die Ringe A¹, A², A³, A⁴ und A⁵ jeweils unabhängig eine trans-Cyclohexan-1,4-diyl-, Cyclohexa-1-en-1,4-diyl-, 1,4-Phenylen-, Bicyclo[1.1.1]pentan-1,3-diyl-, Tetrahydropyran-2,5-diyl-Gruppe oder einen Ring darstellt, dargestellt durch die folgende Formel (1): 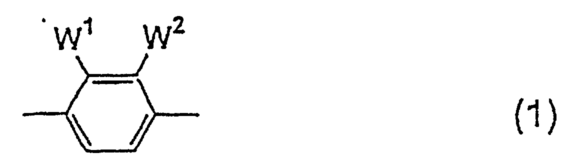 wobei W¹ und W² jeweils unabhängig ein Fluoratom darstellt, mit der Maßgabe, daß mindestens einer dieser Ringe A¹, A², A³, A⁴ und A⁵ einen Tetrahydropyran-2,5-diyl-Ring darstellt und mindestens einer dieser einen durch die Formel (1) dargestellten Ring darstellt, daß die diese Ringe bildenden Kohlenstoffatome mit einem Stickstoff-, Sauerstoff- oder Schwefelatom substituiert sein können und daß jedes in den Ringen vorliegende Wasserstoffatom mit einem Halogenatom oder einer Cyangruppe substituiert sein kann, a¹, a², a³, a⁴ und a⁵ jeweils unabhängig 0 oder 1 darstellt, mit der Maßgabe, daß sie der Beziehung 4 ≥ a¹+a²+a³+a⁴+a⁵ ≥ 2 genügen, und wobei jedes die Verbindung bildende Atom mit seinem Isotop substituiert sein kann.

2. Flüssigkristallverbindung nach Anspruch 1, wobei der durch die Formel (1) dargestellte Ring 2,3-Difluorbenzol-1,4-diyl ist.

3. Flüssigkristallzusammensetzung, welche mindestens zwei Komponenten, die mindestens eine Flüssigkristallverbindung nach Anspruch 1 oder 2 beinhalten, umfaßt.

4. Flüssigkristallzusammensetzung, umfassend mindestens eine Flüssigkristallverbindung nach Anspruch 1 oder 2 als eine erste Komponente und mindestens eine Verbindung, ausgewählt aus der Gruppe, bestehend aus denen, die durch die folgenden allgemeinen Formeln (3), (4) und (5) dargestellt sind, als eine zweite Komponente: 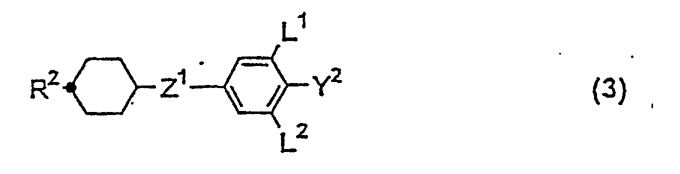 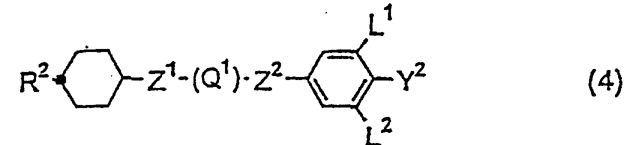 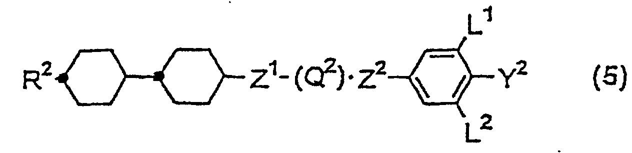 wobei R² eine Alkylgruppe mit 1 bis 10 Kohlenstoffatomen darstellt, mit der Maßgabe, daß mindestens eine der in der Alkylgruppe vorliegenden Methylengruppen, die einander nicht benachbart sind, mit einem Sauerstoffatom oder -CH=CH- substituiert sein kann, und daß mindestens eines der in der Alkylgruppe vorliegenden Wasserstoffatome mit einem Fluoratom substituiert sein kann, Y² ein Fluor- oder Chloratom, -OCF₃, -OCF₂H, -CF₃, -CF₂H, -CFH₂, -OCF₂CF₂H oder -OCF₂CFHCF₃ darstellt, L¹ und L² jeweils unabhängig ein Wasserstoff- oder Fluoratom darstellt, Z¹ und Z² jeweils unabhängig -(CH₂)₂-, -(CH₂)₄-, -COO-, -CF₂O-, -OCF₂-, -CH=CH- oder eine Einfachbindung darstellt, der Ring Q¹ eine trans-Cyclohexan-1,4-diyl- oder 1,3-Dioxan-2,5-diylgruppe oder eine 1,4-Phenylengruppe, deren Wasserstoffatom mit einem Fluoratom substituiert sein kann, darstellt, der Ring Q² eine trans-Cyclohexan-1,4-diyl- oder eine 1,4-Phenylengruppe, deren Wasserstoffatom mit einem Fluoratom substituiert sein kann, darstellt, und wobei jedes die Verbindung bildende Atom mit seinem Isotop substituiert sein kann.

5. Flüssigkristallzusammensetzung, umfassend mindestens eine Flüssigkristallverbindung nach Anspruch 1 oder 2 als eine erste Komponente und mindestens eine Verbindung, ausgewählt aus der Gruppe, bestehend aus denen, dargestellt durch die folgenden allgemeinen Formeln (6) und (7), als eine zweite Komponente: 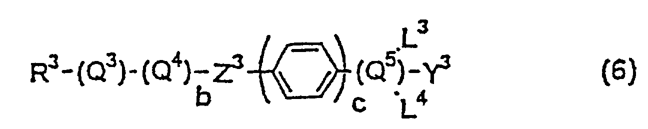 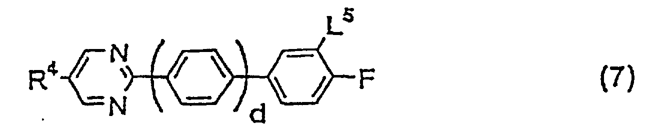 wobei R³ und R⁴ jeweils unabhängig eine Alkylgruppe mit 1 bis 10 Kohlenstoffatomen darstellt, mit der Maßgabe, daß mindestens eine der in der Alkylgruppe vorliegenden Methylengruppen, welche nicht miteinander benachbart sind, mit einem Sauerstoffatom oder-CH=CH- substituiert sein kann, und daß mindestens eines der in der Alkylgruppe vorliegenden Wasserstoffatome mit einem Fluoratom substituiert sein kann, Y³ eine Cyangruppe oder -C≡C-CN darstellt, der Ring Q³ eine trans-Cyclohexan-1,4-diyl-, 1,4-Phenylen-, 1,3-Dioxan-2,5-diyl- oder Pyrimidin-2,5-diylgruppe darstellt, der Ring Q⁴ eine trans-Cyclohexan-1,4-diyl- oder Pyrimidin-2,5-diylgruppe oder eine 1,4-Phenylengruppe, deren Wasserstoffatom mit einem Fluoratom substituiert sein kann, darstellt, der Ring Q⁵ eine trans-Cyclohexan-1,4-diyl- oder 1,4-Phenylengruppe darstellt, Z³ -(CH₂)₂-, -COO- oder eine Einfachbindung darstellt, L³, L⁴ und L⁵ jeweils unabhängig ein Wasserstoff- oder Fluoratom darstellt, b, c und d jeweils unabhängig 0 oder 1 darstellt, und wobei jedes die Verbindung bildende Atom mit seinem Isotop substituiert sein kann.

6. Flüssigkristallzusammensetzung, umfassend mindestens eine Flüssigkristallverbindung nach Anspruch 1 oder 2 als eine erste Komponente, mindestens eine Verbindung, ausgewählt aus der Gruppe, bestehend aus denen, dargestellt durch die allgemeinen Formeln (3), (4) und (5), als eine zweite Komponente und mindestens eine Verbindung, ausgewählt aus der Gruppe, bestehend aus denen, dargestellt durch die folgenden allgemeinen Formeln (8), (9) und (10), als eine dritte Komponente: 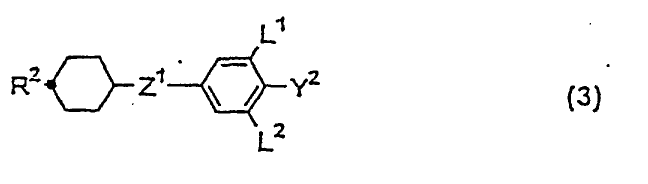 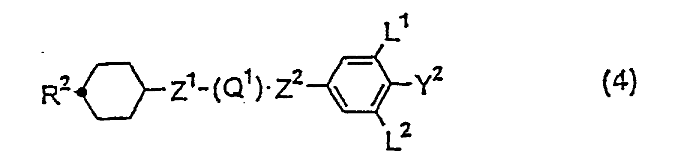 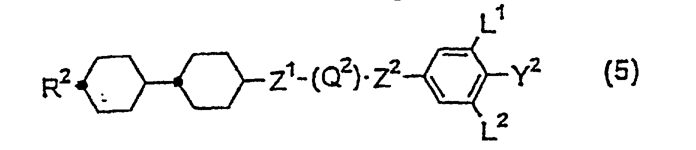
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9) 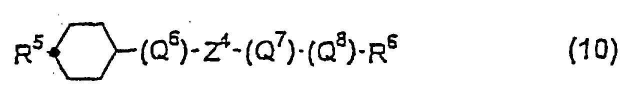
wobei R⁵ und R⁶ jeweils unabhängig eine Alkylgruppe mit 1 bis 10 Kohlenstoffatomen darstellt, mit der Maßgabe, daß mindestens eine der in der Alkylgruppe vorliegenden Methylengruppen, welche nicht miteinander benachbart sind, mit einem Sauerstoffatom oder -CH=CH- substituiert sein kann, und daß mindestens eines der in der Alkylgruppe vorliegenden Wasserstoffatome mit einem Fluoratom substituiert sein kann, die Ringe Q⁶, Q⁷ und Q⁸ jeweils unabhängig eine trans-Cyclohexan-1,4-diyl- oder Pyrimidin-2,5-diylgruppe oder eine 1,4-Phenylengruppe, deren Wasserstoffatom mit einem Fluoratom substituiert sein kann, darstellt, Z⁴ und Z⁵ jeweils unabhängig -C≡C-, -COO-, -(CH₂)₂-, -CH=CH- oder eine Einfachbindung darstellt, und wobei jedes die Verbindung bildende Atom mit seinem Isotop substituiert sein kann.

7. Flüssigkristallzusammensetzung, umfassend mindestens eine Flüssigkristallverbindung nach Anspruch 1 oder 2 als eine erste Komponente und mindestens eine Verbindung, ausgewählt aus der Gruppe, bestehend aus denen, dargestellt durch die folgenden allgemeinen Formeln (11), (12) und (13), als eine zweite Komponente: 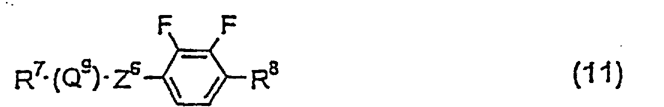 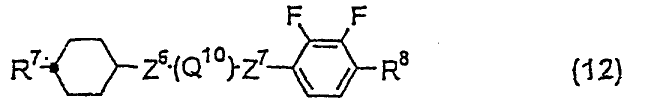 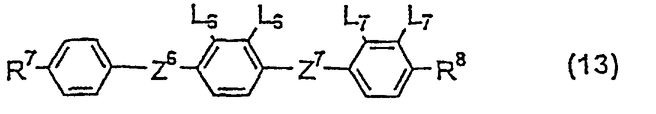 wobei R⁷ und R⁸ jeweils unabhängig eine Alkylgruppe mit 1 bis 10 Kohlenstoffatomen darstellt, mit der Maßgabe, daß mindestens eine der in der Alkylgruppe vorliegenden Methylengruppen, welche nicht miteinander benachbart sind, mit einem Sauerstoffatom oder -CH=CH- substituiert sein kann, und daß mindestens eines der in der Alkylgruppe vorliegenden Wasserstoffatome mit einem Fluoratom substituiert sein kann, die Ringe Q⁹ und Q¹⁰ jeweils unabhängig eine trans-Cyclohexan-1,4-diyl- oder 1,4-Phenylengruppe darstellt, L⁶ und L⁷ jeweils unabhängig ein Wasserstoffoder Fluoratom darstellt, mit der Maßgabe, daß L⁶ und L⁷ nicht gleichzeitig ein Wasserstoffatom darstellen, Z⁶ und Z⁷ jeweils unabhängig -(CH₂)₂-, -COO- oder eine Einfachbindung darstellt, und wobei jedes die Verbindung bildende Atom mit seinem Isotop substituiert sein kann.

8. Flüssigkristallzusammensetzung, umfassend mindestens eine Flüssigkristallverbindung nach Anspruch 1 oder 2 als eine erste Komponente, mindestens eine Verbindung, ausgewählt aus der Gruppe, bestehend aus denen, dargestellt durch die allgemeinen Formeln (8), (9) und (10), als eine zweite Komponente und mindestens eine Verbindung, ausgewählt aus der Gruppe, bestehend aus denen, dargestellt durch die allgemeinen Fomeln (11), (12) und (13), als eine dritte Komponente:
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

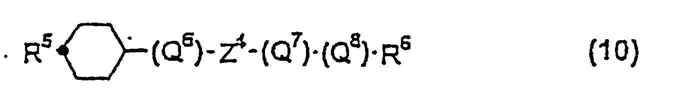

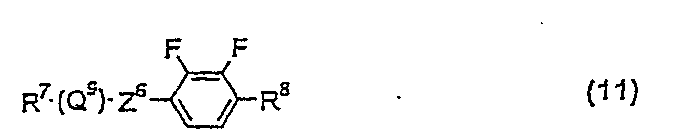

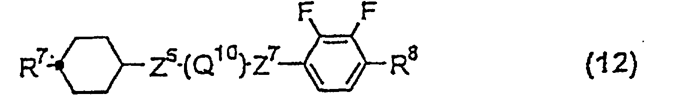

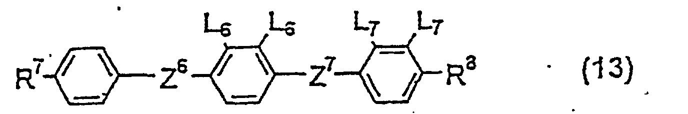

9. Flüssigkristallzusammensetzung, umfassend mindestens eine Flüssigkristallverbindung nach Anspruch 1 oder 2 als eine erste Komponente, mindestens eine Verbindung, ausgewählt aus der Gruppe, bestehend aus denen, dargestellt durch die allgemeinen Formeln (6) und (7) als eine zweite Komponente und mindestens eine Verbindung, ausgewählt aus der Gruppe, bestehend aus denen, dargestellt durch die allgemeinen Formeln (8), (9) und (10), als eine dritte Komponente: 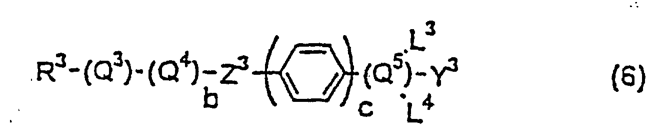 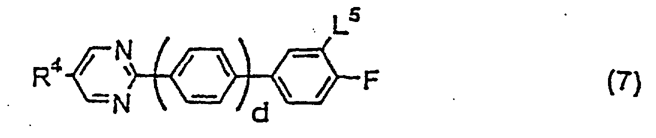
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

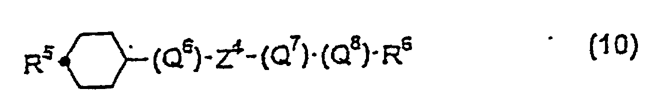

10. Flüssigkristallzusammensetzung, umfassend mindestens eine Flüssigkristallverbindung nach Anspruch 1 oder 2 als eine erste Komponente, mindestens eine Verbindung, ausgewählt aus der Gruppe, bestehend aus denen, dargestellt durch die allgemeinen Formeln (3), (4) und (5), als eine zweite Komponente, mindestens eine Verbindung, ausgewählt aus der Gruppe, bestehend aus denen, dargestellt durch die allgemeinen Formeln (6) und (7) als eine dritte Komponente und mindestens eine Verbindung, ausgewählt aus der Gruppe, bestehend aus denen, dargestellt durch die allgemeinen Formeln (8), (9) und (10), als eine vierte Komponente: 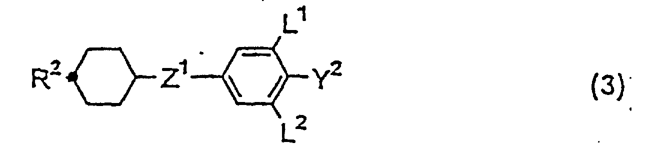 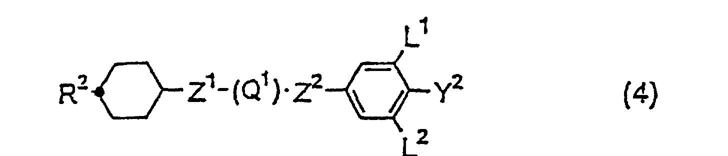 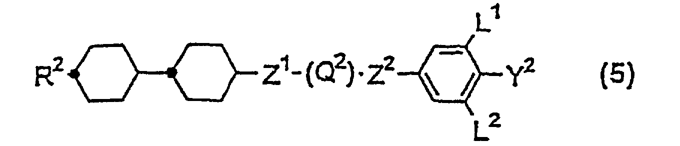 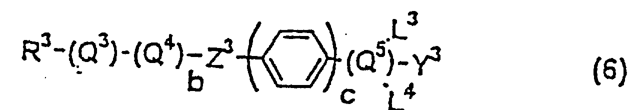 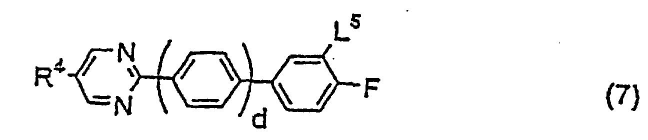
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

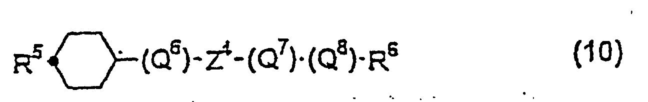

11. Flüssigkristallzusammensetzung, welche mindestens eine optisch aktive Verbindung zusätzlich zu einer der Flüssigkristallzusammensetzungen nach einem der Ansprüche 3 bis 10 umfaßt.

12. Flüssigkristallanzeigegerät, konstituiert durch die Verwendung einer der Flüssigkristallzusammensetzungen nach einem der Ansprüche 3 bis 11.

## Revendications

1. Composé du type cristal liquide représenté par la formule générale (2) suivante :
R¹-(A¹-X¹)ₐ₁-(A²-X²)ₐ₂-(A³-X³)ₐ₃-(A⁴-X⁴)ₐ₄-(A⁵) ₐ₅-Y¹ (2)
dans laquelle R¹ et Y¹ représentent chacun, indépendamment, un groupe alkyle ayant 1 à 20 atomes de carbone, un atome d'hydrogène, un atome d'halogène, un groupe cyano, un résidu cyanate, un groupe isocyano ou un résidu isothiocyanate, sous réserve qu'au moins un des groupes méthylène présents dans le groupe alkyle, qui ne sont pas adjacents les uns aux autres, puisse être substitué avec un atome d'oxygène, de soufre ou d'azote, un groupe -C≡C-, un groupe silylène dans lequel au moins un atome d'hydrogène peut être substitué avec un groupe alkyle ayant 1 à 10 atomes de carbone, ou un groupe vinylène, ou qu'au moins un atome d'hydrogène du groupe alkyle puisse être substitué avec un atome de fluor ou de chlore ou bien que ces groupes alkyle puissent être des groupes alkyle optiquement actifs ; X¹, X², X³ et X⁴ représentent chacun, indépendamment, une liaison simple, un groupe -(CH₂)₂-, -CH=CH-, -C≡C-, -COO-, -OCO-, -CH₂O-, -OCH₂-, -(CH₂)₄-, - (CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, - (CH₂)₂COO-, -OCO(CH₂)₂-, -CH=CHCOO-, -OCOCH=CH-, -CH=CHC≡C- ou -C≡CCH=CH- ; les noyaux A¹, A², A³, A⁴ et A⁵ représentent chacun, indépendamment, un groupe trans-cyclohexane-1,4-diyle, cyclohexa-1-ène-1,4-diyle, 1,4-phénylène, bicyclo[1.1.1]pentane-1,3-diyle ou tétrahydropyranne-2,5-diyle, ou un noyau représenté par la formule (1) suivante : 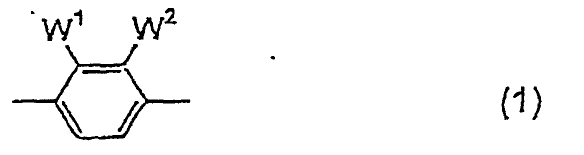 dans laquelle W¹ et W² représentent chacun, indépendamment, un atome de fluor, sous réserve qu'au moins un de ces noyaux A¹, A², A³, A⁴ et A⁵ représente un noyau tétrahydro-pyranne-2,5-diyle et au moins un d'entre eux représente un noyau représenté par la formule (1), que les atomes de carbone constituant ces noyaux puissent être substitués avec un atome d'azote, d'oxygène ou de soufre et que chaque atome d'hydrogène présent sur les noyaux puisse être substitué avec un atome d'halogène ou un groupe cyano ; a¹, a², a³, a⁴ et a⁵ ont chacun, indépendamment, la valeur 0 ou 1, sous réserve qu'ils satisfassent la relation : 4 ≥ a¹+a²+a³+a⁴+a⁵ ≥ 2 ; et chaque atome constituant le composé peut être substitué par son isotope.

2. Composé du type cristal liquide suivant la revendication 1, dans lequel le noyau représenté par la formule (1) est un noyau 2,3-difluorobenzène-1,4-diyle.

3. Composition de cristal liquide comprenant au moins deux constituants comprenant au moins un composé du type cristal liquide répondant à la définition suivant la revendication 1 ou 2.

4. Composition de cristal liquide comprenant au moins un composé du type cristal liquide répondant à la définition suivant la revendication 1 ou 2 comme premier constituant et au moins un composé choisi dans le groupe consistant en ceux représentés par les formules générales (3), (4) et (5) suivantes comme second constituant : 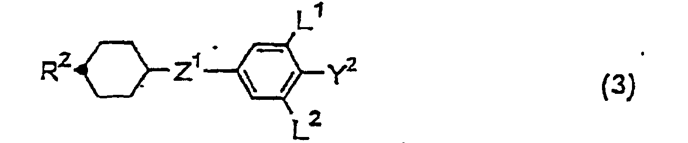 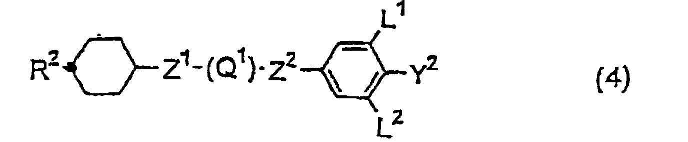 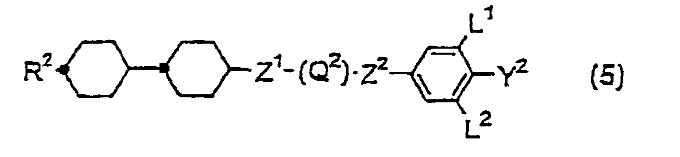 formules dans lesquelles R² représente un groupe alkyle ayant 1 à 10 atomes de carbone, sous réserve qu'au moins un des groupes méthylène présents dans le groupe alkyle, qui ne sont pas adjacents les uns aux autres, puisse être substitué avec un atome d'oxygène ou un groupe -CH=CH- et qu'au moins un des atomes d'hydrogène présents dans le groupe alkyle puisse être substitué avec un atome de fluor ; Y² représente un atome de fluor ou de chlore, un groupe -OCF₃, -OCF₂H, -CF₃, -CF₂H, -CFH₂, -OCF₂CF₂H ou -OCF₂CFHCF₃ ; L¹ et L² représentent chacun, indépendamment, un atome d'hydrogène ou de fluor ; Z¹ et Z² représentent chacun, indépendamment, un groupe -(CH₂)₂-, -(CH₂)₄-, -COO-, -CF₂O-, -OCF₂-, -CH=CH- ou une liaison simple ; le noyau Q¹ représente un groupe trans-cyclohexane-1,4-diyle ou 1,3-dioxanne-2,5-diyle, ou un groupe 1,4-phénylène dont un atome d'hydrogène peut être substitué par un atome de fluor ; le noyau Q² représente un groupe trans-cyclohexane-1,4-diyle, ou un groupe 1,4-phénylène dont un atome d'hydrogène peut être substitué par un atome de fluor ; et chaque atome constituant le composé peut être substitué par son isotope.

5. Composition de cristal liquide comprenant au moins un composé du type cristal liquide répondant à la définition suivant la revendication 1 ou 2 comme premier constituant et au moins un composé choisi dans le groupe consistant en ceux représentés par les formules générales (6) et (7) suivantes comme second constituant : 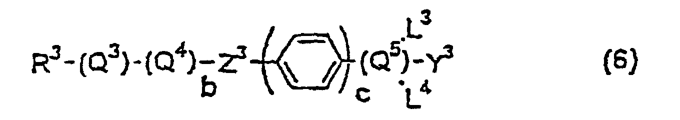 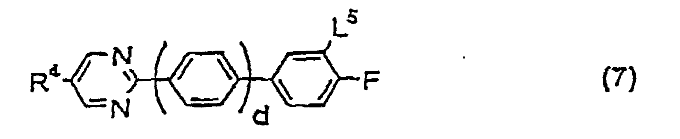 formules dans lesquelles R³ et R⁴ représentent chacun, indépendamment, un groupe alkyle ayant 1 à 10 atomes de carbone, sous réserve qu'au moins un des groupes méthylène présents dans le groupe alkyle, qui ne sont pas adjacents les uns aux autres, puisse être substitué avec un atome d'oxygène ou un groupe -CH=CH- et qu'au moins un des atomes d'hydrogène présents dans le groupe alkyle puisse être substitué par un atome de fluor ; Y³ représente un groupe cyano ou -C≡C-CN ; le noyau Q³ représente un groupe transcyclohexane-1,4-diyle, 1,4-phénylène, 1,3-dioxanne-2,5-diyle ou pyrimidine-2,5-diyle ; le noyau Q⁴ représente un groupe trans-cyclohexane-1,4-diyle ou pyrimidine-2,5-diyle, et un groupe 1,4-phénylène dont un atome d'hydrogène peut être substitué par un atome de fluor ; le noyau Q⁵ représente un groupe trans-cyclohexane-1,4-diyle ou 1,4-phénylène ; Z³ représente un groupe -(CH₂)₂-, -COO- ou une liaison simple ; L³, L⁴ et L⁵ représentent chacun, indépendamment, un atome d'hydrogène ou de fluor ; b, c et d sont chacun égaux, indépendamment, à 0 ou 1 ; et chaque atome constituant le composé peut être substitué par son isotope.

6. Composition de cristal liquide comprenant au moins un composé du type cristal liquide répondant à la définition suivant la revendication 1 ou 2 comme premier constituant ; au moins un composé choisi dans le groupe consistant en ceux représentés par les formules générales (3), (4) et (5) comme deuxième constituant ; et au moins un composé choisi dans le groupe consistant en ceux représentés par les formules générales (8), (9) et (10) suivantes comme troisième constituant : 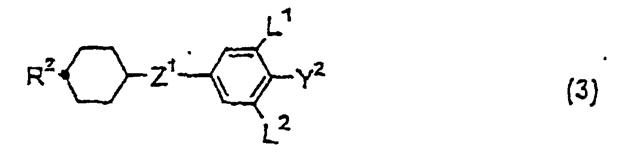 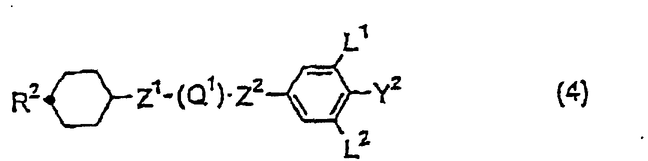 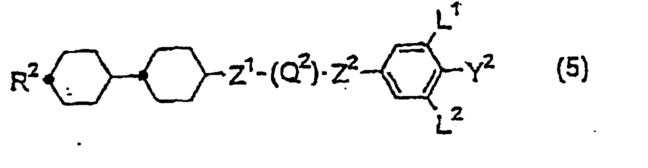
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9) 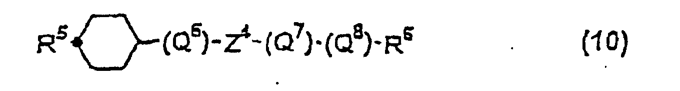
formules dans lesquelles R⁵ et R⁶ représentent chacun, indépendamment, un groupe alkyle ayant 1 à 10 atomes de carbone, sous réserve qu'au moins un des groupes méthylène présents dans le groupe alkyle, qui ne sont pas adjacents les uns aux autres, puisse être substitué avec un atome d'oxygène ou un groupe -CH=CH- et qu'au moins un des atomes d'hydrogène présents dans le groupe alkyle puisse être substitué par un atome de fluor ; les noyaux Q⁶, Q⁷ et Q⁸ représentent chacun, indépendamment, un groupe transcyclohexane-1,4-diyle ou pyrimidine-2,5-diyle, ou un groupe 1,4-phénylène dont un atome d'hydrogène peut être substitué par un atome de fluor ; Z⁴ et Z⁵ représentent chacun, indépendamment, un groupe -C≡C-, -COO-, -(CH₂)₂-, -CH=CH- ou une liaison simple ; et chaque atome constituant le composé peut être substitué par son isotope.

7. Composition de cristal liquide comprenant au moins un composé du type cristal liquide répondant à la définition suivant la revendication 1 ou 2 comme premier constituant et au moins un composé choisi dans le groupe consistant en ceux représentés par les formules générales (11), (12) et (13) suivantes comme second constituant : 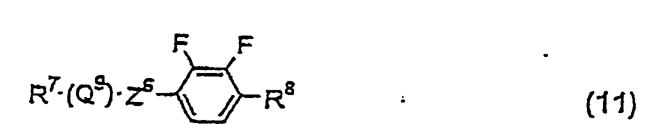 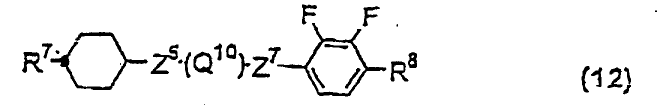 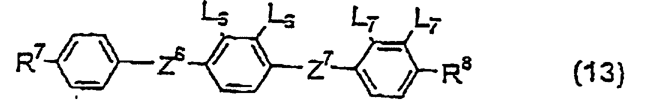 formules dans lesquelles R⁷ et R⁸ représentent chacun, indépendamment, un groupe alkyle ayant 1 à 10 atomes de carbone, sous réserve qu'au moins un des groupes méthylène présents dans le groupe alkyle, qui ne sont pas adjacents les uns aux autres, puisse être substitué avec un atome d'oxygène ou un groupe -CH=CH- et qu'au moins un des atomes d'hydrogène présents dans le groupe alkyle puisse être substitué par un atome de fluor ; les noyaux Q⁹ et Q¹⁰ représentent chacun, indépendamment, un groupe transcyclohexane-1,4-diyle ou 1,4-phénylène ; L⁶ et L⁷ représentent chacun, indépendamment, un atome d'hydrogène ou de fluor, sous réserve que L⁶ et L⁷ ne représentent pas simultanément un atome d'hydrogène ; Z⁶ et Z⁷ représentent chacun, indépendamment, un groupe -(CH₂)₂-, -COO- ou une liaison simple ; et chaque atome constituant le composé peut être substitué par son isotope.

8. Composition de cristal liquide comprenant au moins un composé du type cristal liquide répondant à la définition suivant la revendication 1 ou 2 comme premier constituant ; au moins un composé choisi dans le groupe consistant en ceux représentés par les formules générales (8), (9) et (10) comme deuxième constituant ; et au moins un composé choisi dans le groupe consistant en ceux représentés par les formules générales (11), (12) et (13) comme troisième constituant :
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

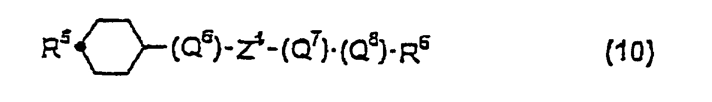

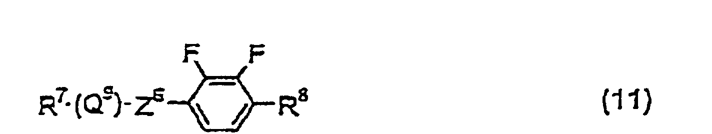

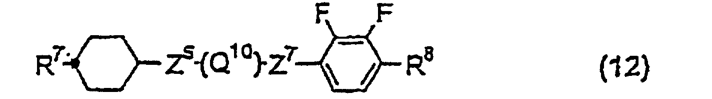

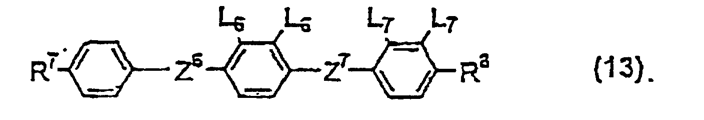

9. Composition de cristal liquide comprenant au moins un composé du type cristal liquide répondant à la définition suivant la revendication 1 ou 2 comme premier constituant ; au moins un composé choisi dans le groupe consistant en ceux représentés par les formules générales (6) et (7) comme deuxième constituant ; et au moins un composé choisi dans le groupe consistant en ceux représentés par les formules générales (8), (9) et (10) comme troisième constituant : 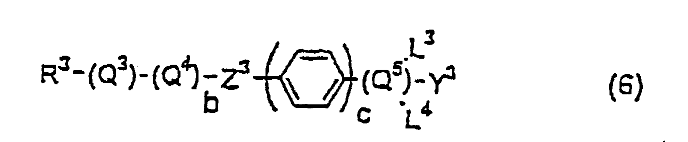 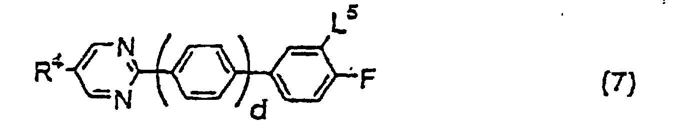
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

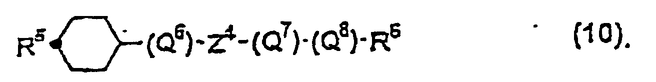

10. Composition de cristal liquide comprenant au moins un composé du type cristal liquide répondant à la définition suivant la revendication 1 ou 2 comme premier constituant ; au moins un composé choisi dans le groupe consistant en ceux représentés par les formules générales (3), (4) et (5) comme deuxième constituant ; au moins un composé choisi dans le groupe consistant en ceux représentés par les formules générales (6) et (7) comme troisième constituant ; et au moins un composé choisi dans le groupe consistant en ceux représentés par les formules générales (8), (9) et (10) comme quatrième constituant : 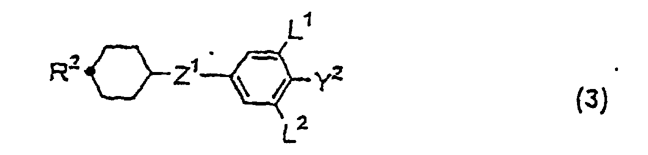 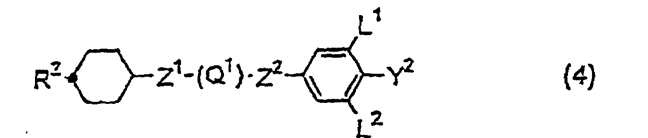 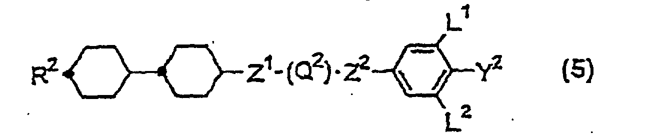 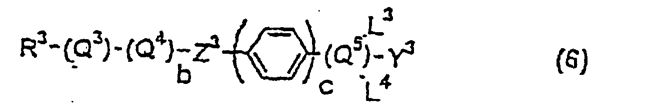 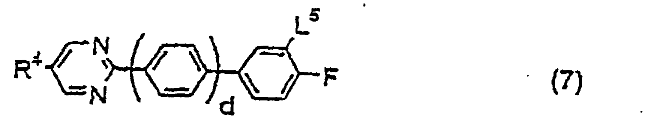
R⁵·(Q⁶)-Z⁴·(Q⁷)-Z⁵―R⁶ (8)
R⁵·(Q⁶)-Z⁴·(Q⁷)-Z⁵-(Q⁸)·R⁶ (9)

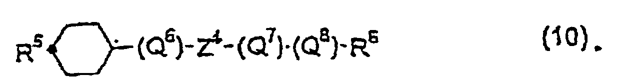

11. Composition de cristal liquide comprenant au moins un composé optiquement actif en plus d'une des compositions de cristaux liquides répondant aux définitions suivant l'une quelconque des revendications 3 à 10.

12. Dispositif d'affichage à cristaux liquides constitué par l'utilisation d'une des compositions de cristaux liquides répondant aux définitions suivant l'une quelconque des revendications 3 à 11.
